# EUROPÄISCHE PATENTSCHRIFT

(11) **EP 2 513 293 B1**
(45) Veröffentlichungstag und Bekanntmachung des Hinweises auf die Patenterteilung: **12.11.2014**
(21) Anmeldenummer: 10798777.8
(22) Anmeldetag: 17.12.2010
(51) Int. Cl.: C07K 16/00, C12P 21/02, C12N 5/00, C12N 5/071

(54) **METHODE ZUR OPTIMIERUNG EINES BIOPHARMAZEUTISCHEN PRODUKTIONSPROZESSES**
METHOD FOR OPTIMISING A BIOPHARMACEUTICAL PRODUCTION PROCESS
PROCÉDÉ D'OPTIMISATION D'UN PROCESSUS DE PRODUCTION BIOPHARMACEUTIQUE

(30) Priorität: 18.12.2009 EP 09179954; 28.04.2010 EP 10161272
(43) Veröffentlichungstag der Anmeldung: 24.10.2012
(73) Patentinhaber: Boehringer Ingelheim International GmbH, 55216 Ingelheim am Rhein (DE)
(72) Erfinder: SCHLATTER, Stefan, 55216 Ingelheim am Rhein (DE); SAUTER, Christian, 55216 Ingelheim am Rhein (DE); PROBST, Eugen, 55216 Ingelheim am Rhein (DE); WIEDEMANN, Franz, 55216 Ingelheim am Rhein (DE); GUELCH, Carina, 55216 Ingelheim am Rhein (DE)
(74) Vertreter: Simon, Elke Anna Maria
(86) Internationale Anmeldenummer: PCT/EP2010/070042
(87) Internationale Veröffentlichungsnummer: WO 2011/073377

(56) Entgegenhaltungen:
- WO-A1-01/52647
- WO-A1-91/10726
- WO-A1-97/38090
- WO-A1-2010/017338
- US-B1- 6 338 964
- WILLIAMSON J D ET AL: "Use of a new buffer in the culture of animal cells.", THE JOURNAL OF GENERAL VIROLOGY MAR 1968 LNKD- PUBMED:5654767, Bd. 2, Nr. 2, März 1968 (1968-03), Seiten 309-312, XP002601061, ISSN: 0022-1317
- HASEGAWA K ET AL: "Culturing method providing algae contg docosa-hexa:enoic acid - comprises culturing in sodium chloride-enriched medium", WPI / THOMSON,, Bd. 1995, Nr. 20, 20. März 1995 (1995-03-20), XP002589921, & JP 7 075557 A (NISSHIN OIL MILLS LTD) 20. März 1995 (1995-03-20)
- MATSUTANI Y ET AL: "Serum-free synthetic medium used for cell culture - comprising medium contg. mixt. of eagle mem and rpm11640, and additives, with their osmotic pressure adjusted", WPI / THOMSON,, Bd. 1986, Nr. 11, 4. Februar 1986 (1986-02-04), XP002589922,
- ZHU MARIE M ET AL: "Effects of elevated pCO2 and osmolality on growth of CHO cells and production of antibody-fusion protein B1: a case study", BIOTECHNOLOGY PROGRESS 2005 JAN-FEB,, Bd. 21, Nr. 1, 1. Januar 2005 (2005-01-01) , Seiten 70-77, XP002536931,
- LAIRD W ET AL: "Rapid, direct tissue culture test for toxigenicity of Corynebacterium diphtheriae.", APPLIED MICROBIOLOGY MAY 1973 LNKD- PUBMED:4197640, Bd. 25, Nr. 5, Mai 1973 (1973-05), Seiten 709-712, XP002601062, ISSN: 0003-6919
- STEFAN GNOTH ET AL: "Control of cultivation processes for recombinant protein production: a review", BIOPROCESS AND BIOSYSTEMS ENGINEERING, SPRINGER, BERLIN, DE, Bd. 31, Nr. 1, 5. Oktober 2007 (2007-10-05), Seiten 21-39, XP019564177, ISSN: 1615-7605
- ROBINSON D K ET AL: "OPTIMIZATION OF A FED-BATCH PROCESS FOR PRODUCTION OF A RECOMBINANT ANTIBODY", ANNALS OF THE NEW YORK ACADEMY OF SCIENCES, NEW YORK ACADEMY OF SCIENCES, NEW YORK, NY, US, Bd. 745, 30. November 1994 (1994-11-30), Seiten 285-296, XP009021037, ISSN: 0077-8923

## Beschreibung

### HINTERGRUND DER ERFINDUNG

### TECHNISCHES GEBIET

Die Erfindung betrifft das Gebiet der biopharmazeutischen Prozessentwicklung mit eukaryotischen Zellen, welche rekombinantes Protein von Interesse produzieren. Insbesondere betrifft die Erfindung eine Methode zur Optimierung der Prozessführung welche den Einsatz eines Na₂CO₃ und NaHCO₃ freien Mediums in Kombination mit einer pCO₂-Regelung umfasst.

### HINTERGRUND

Die biopharmazeutischen Prozessentwicklung steht vor der Herausforderung, immer höhere Titer bereitzustellen für die Produktion von therapeutischen Proteinen, insbesondere Antikörpern für klinische (Toxikologie-) Studien mit engen Zeitvorgaben oder die Marktversorgung.

Im Rahmen dieser Zeitvorgaben müssen Expressionssysteme entworfen werden, müssen stabile Produktionszellklone für die Produktion generiert und ausgewählt werden (z.B. CHO Zellen, Hybridome, BHK oder NSO Zellen), müssen skalierbare biopharmazeutische Produktionsprozesse entworfen werden umfassend Medienoptimierung und Prozesskontrolle. All das muss adressiert werden, damit die spezifische Produktivität (=spezifische Produktbildungsrate) und die erreichten Titer (Produktausbeute) maximiert werden um die Prozesse robust und reproduzierbar in unterschiedlich gross dimensionierten Anlagen fahren zu können. In den letzten Jahren wurden enorme Steigerungen bei Produkttiter in rekombinanten eukaryotischen Zellsystemen erreicht. So wurden beispielsweise Produktkonzentrationen von über 5g Immunoglobulin/L in Chinese Hamster Ovary (CHO) Zellen erreicht. Fortschritte in der Molekularbiologie und der Zellbiologie umfassen genetische Zelllinienentwicklung und -veränderungen, Medienentwicklung, und "In-Prozess" Kontrollstrategien wie Nährstoffzugabe ("Feed") haben dies u.a. ermöglicht. Dennoch erreichen die Produktivitäten von eukaryotischen Zellsystemen, insbesondere Säugerzellsystemen, nicht diejenigen von prokaryotischen Zellsystemen und daher bleibt die weitere Optimierung insbesondere der Prozesskontrolle bei der Fermentation und die Optimierung des Fermentationsmediums eine ständige Herausforderung.

Eine spezielle Herausforderung ist die Kontrolle des oxidativen Stoffwechsels.

Alle Zellen produzieren im Rahmen des oxidativen Stoffwechsels CO₂ und benötigen HCO₃⁻ für eine Vielzahl gekoppelter Transportprozesse. Erhöhte CO₂-Gehalte, die z.B. bei starkem Wachstum auftreten können, erniedrigen den pH-Wert, was durch erhöhten Natriumhydrogencarbonatgehalt neutralisiert werden kann. Damit ist Natriumhydrogencarbonat sowohl Puffersubstanz als auch essentieller Nahrungsbestandteil. Natriumhydrogencarbonat wird u.a. im Lehrbuch Lindl, Toni; Gstraunthaler Gerhard (2002): Zell- und Gewebekultur, 5. Auflage, Spektrum Akademischer Verlag Heidelberg, S. 93 Punkt 4.4.3 als essentieller Nahrungsbestandteil beschrieben oder auch in Ling, C. T. et. al (1968): Chemically characterized concentrated corodies for continuous cell culture (The 7C's culture media), in Experimental Cell Research Nr. 52, S. 469-489.

Hydrogencarbonat spielt des Weiteren eine Rolle im Zitronensäure-Zyklus, bei der pH-Regulation des gesamten Körpers, sowie einzelner Zellen und deren Volumen-Regulation. Die Membranen von Säugerzellen beinhalten Transportproteine für Hydrogencarbonat, um den Transmembrantransport zu erleichtern. Während CO₂ in der Lage ist die Lipiddoppelschicht per Diffusion zu durchdringen, ist HCO₃⁻ geladen und durchtritt die Membran nur mit Hilfe von spezifischen Transport-Proteinen. Auf Grund der Säure-Base-Eigenschaften von CO₂/HCO₃⁻, resultiert der CO₂-Ausstoß aus der Zelle in einer Alkalisierung der Zelle während HCO₃⁻ -Ausfluss die Zelle ansäuert (Casey Joseph. R. (2006): Why bicarbonate? in Biochem. Cell Biol. Nr. 84, S. 930-939).

Die US6338964 offenbart ein Verfahren zur Kultivierung von eukaryontischen Wirtszellen z.B CHO, BHK, HeLa), welche rekombinantes Protein produzieren, in einem Zellkulturmedium, welches eine Konzentration von 0.01 bis 1g/1 NaHCO 3 3) bzw. von 0 bis 0.01 g/l NaHCO3 und TES al (0.19 - 1.9mol/INaHCOs Pufferkomponente enthält. Während des Verfahrens, das in einem Rührkesselfermentor durchgeführt wird, wird der pH-Wert über eine nicht CO₂-bildende Lauge wie z.B NaOH und der pCO₂ mittels Luftzufuhr oder Drehzahl des Rührers reguliert.

Williamson et al. "Use of a new Buffer in the culture of animal cells."THE JOURNAL OF GENERAL VIROLOGY, March 1968, Bd. 2, Nr. 2, März 1968 (1968-03) , Seiten 309-312, ISSN: 0022-1317 offenbart Zellkulturmedien, die TES oder HEPES als Pufferkomponente beinhalten: den käuflichen Medien Eagle's MEM oder Medium 199 werden TES oder HEPES mit einer Endkonzentration von 28mM zugesetzt und mit dem Wachstum von Zelllinien in Medien, die mit Bicarbonat + CO₂, TES oder HEPES gepuffert sind verglichen.

Die WO 91/10726 offenbart ein Zellkulturmedium, das 75mg/1 NaHCO₃ (0.89mmol/l) und 4320mg/l Sod-ß (14mmol/l) als Pufferkomponente enthält.

Allerdings offenbart keines der Dokumente im Stand der Technik eine Methode bzw. ein Verfahren sowie ein Zellkulturmedium gemäß der vorliegenden Erfindung, welches keine HCO₃⁻ bzw. CO₃²⁻ Ionen enthält/verwendet und als Pufferkomponente Sodium-β-glycerophosphat-pentahydrat (C₃H₇Na₂O₆P x 5 H₂O, Sod-ß) enthält/verwendet.

### Zusammenfassung der Erfindung

Um das Ziel, CO₂ im Fermentationsprozess vollständig regulieren zu können, zu erreichen, müssen HCO₃⁻ bzw. CO₃²⁻ Ionen z.B. stammend aus Natriumhydrogencarbonat Puffer durch eine alternative Puffersubstanz ersetzt werden. Aktuell kann, wie in folgender chemischer Gleichung gezeigt, bei der Reaktion mit Milchsäure (MS) CO₂ entstehen:

NaHCO_{3 (aq)} + MS _{(aq)} → Na-Laktat _{(aq)} + CO_{2 (aq)} +H₂0 (Gl. 1)

Auch die Regelung des pH-Wertes erfolgt aktuell mit Na₂CO₃-Lösung welche, wie in Gl. 2 dargestellt, zu NaHCO₃ zerfallen kann. Das Produkt NaHCO₃ aus Gl.2 wird wiederum zum Edukt in Gl. 1.

Na₂CO_{3 (aq)} + H₂O → NaHCO_{3 (aq)} + OH⁻ + Na⁺ (Gl. 2)

Wie oben beschrieben, können Zellen nicht ohne HCO₃⁻ existieren, da es obligat für wichtige Zellfunktionen ist. Es erscheint daher unmöglich, auf HCO₃⁻ - aus der Quelle Puffersubstanz NaHCO₃ oder aus der Quelle pH-Regulationsagens Na₂CO₃-Lösung, vollständig zu verzichten. Die Zellen haben jedoch noch weitere Quellen für Bicarbonat:

CO₂ + H₂O ⇆ H₂CO₃ ⇆ HCO₃⁻ + H⁺ (Gl. 3)

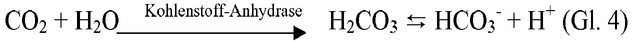

Während Gl. 3 spontan abläuft, z.B. beim Eingasen von CO₂, so ist die Reaktion von Gl. 4 noch zusätzlich durch das intrazelluläre Enzym Kohlenstoff-Anhydrase unterstützt. Die Zelle kann sich also bei Bedarf ihr Bicarbonat selbst aus H₂O (vorhanden in Zellkulturmedium) und CO₂ (wird eingegast oder Nebenprodukt aus Zellzyklus) synthetisieren.

Der erste Schritt auf dem Wege zur pCO₂-Regelung im Fermentationssystem ist die Suche nach alternativen Puffersubstanzen für Natriumhydrogencarbonat, NaHCO₃. Mit dem Standardmedium/ NaHCO₃-haltigen Standardpuffer bildet sich ein Gleichgewicht zwischen CO₂ (gas) und CO_{2 (aq)} aus, welches den pH-Wert der Zellkultur beeinflusst.

Die neue, alternative Puffersubstanz, muss bei vollständigen oder teilweisen Ersatz von HCO₃⁻ bzw. CO₃²⁻ Ionen z.B. stammend aus Natriumhydrogencarbonat eine gute Pufferwirkung zeigen und darf sich nicht negativ auf die Zellkultur auswirken. Auch müssen die Performance und die Produktbildung (Titer) mindestens auf gleich hohem Niveau bleiben. Es könnte z.B. der Fall auftreten, dass die neue Puffersubstanz zwar ein gutes Pufferverhalten zeigt, jedoch toxisch auf die Zelle wirkt oder das extrazellulär sezernierte Produkt schädigt. Ferner könnten unerwünschte Reaktionen mit den weiteren Bestandteilen des Zellkulturmediums auftreten und z.B. die Vitalitätsraten absinken. Eine gesunkene Vitalität bedeutet mehr tote Zellen in der Kultur, welche schädigende Zellinhalte wie z.B. Proteasen an die Fermentationsbrühe abgeben.

Die neue Puffersubstanz sollte in zweiter Priorität kostengünstig sein, keine besonderen Anforderungen an Lagerung und Handhabung stellen und von verschiedenen Lieferanten zu beziehen sein.

Der zweite Schritt/ ein weiterer Schritt auf dem Wege zur pCO₂-Regelung im Fermentationssystem ist ein optimales CO₂ Profil zu etablieren welches den Stoffwechsel der Zellen positiv beeinflusst indem weniger toxische Stoffwechselprodukte gebildet werden (Laktat, Ammonium) und/oder die Zugaben von Säure und Lauge zur pH Regelung minimiert. Dabei wird das optimale Profil der entsprechenden Wachstumsphase angepasst (Anwachsphase, Wachstumsphase, Absterbephase) mit dem Ziel die Anwachsphase zu verkürzen, die Wachstumsphase zu verlängern und die spezifische Produktbildungsrate während der Wachstumsphase zu erhöhen. Das gewählte CO₂ Profil dient somit der gezielten Steuerung des Prozesses und wird in mehrere Regelabschnitte unterteilt: Ein niedriger pCO₂ in der Anwachsphase (<10% tbd, 3-8% bzw. 3-5%, 5% oder 3%) begünstigt ein schnelleres Anwachsen der Zellen, ein mittlerer pCO₂ (<12% tbd, bevorzugt 5-11%) in der Wachstumsphase begünstigt eine hohe Wachstumsrate und besseren Stoffwechsel wodurch weniger Laktat gebildet wird als bei höherem pCO₂, während ein leicht erhöhter bzw. hoher pCO₂ (>5%, >8%, >15% tbd, 5-10%) in der Absterbephase einer Laugezugabe entgegenwirkt und somit zu einer Erniedrigung des osmotischen Drucks und somit zur Verlängerung der Produktivitätsphase beiträgt.

Ein weiterer wichtiger Aspekt ist die Skalierbarkeit und insbesondere die Übertragbarkeit eines Prozesses über die verschiedenen Fermentervolumina hinweg. In herkömmlichen Fermentationssystemen hängt der pCO₂ von den verwendeten Gerätschaften (=Equipment) und dem Maßstab (=scale) ab und nimmt traditionell mit Maßstabsvergrößerung zu. Diesem Phänomen kann nicht entgegengewirkt werden ohne gleichzeitig den Stoffwechsel der Zelle zu verändern, da in herkömmlichen Systemen der pCO₂ mit der Begasung und der pH Regelung gekoppelt ist. Durch Entkopplung der pCO₂-Regelung von pH- und Begasungsregelung (genauer pO₂-Regelung) durch das in der vorliegenden Erfindung beschriebene Puffersystem in Kombination mit der pCO₂-Regelung, lässt sich eine Maßstabsvergrößerung einfach und ohne grundlegende Veränderung der Fermentationscharakteristika (=Performance) durchführen, dabei werden Begasungs- (genauer pO₂-), pH- und pCO₂-Regelung jeweils separat übertragen und sichern somit eine vergleichbare Zellkulturperformance in allen Fermantationsmaßstäben.

Die vorliegende Erfindung stellt eine Methode zur Optimierung eines biopharmazeutischen Produktionsprozesses bereit und umfasst folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponente Sodium-β-Glycerophosphat-pentahydrat (C₃H₇Na₂O₆P x 5 H₂O, Sod-β) enthält,
c) Regulierung des pH-Wertes über eine nicht CO₂-bildende Säure und/oder Lauge,
wobei während des Bioprozesses eine Regulierung des pCO₂ mit CO₂, O₂, N₂ und/oder Luftzufuhr oder mittels der Drehzahl des Rührers durchgeführt wird. In der Anwachsphase kann ein niedriger pCO₂ eingestellt werden. Bevorzugt ist eine Regulierung des pCO₂ mit CO₂ und/ oder N₂.

Bei der geregelten Fermentation im Bioreaktor wird der pH-Wert über das Eingasen von CO₂ und die Zugabe von flüssiger Lauge, z.Bsp. Na₂CO₃ geregelt. Um jedoch den pH-Wert unabhängig von pCO₂ regeln zu können, muss das Puffersystem und dazugehörige pH-Regulierung mit Agenzien erfolgen, welche nicht CO₂ frei setzten. Durch die Elimination von CO₂-bildendem Puffer und Säure/Base wird eine unabhängige Regelung von DO (dissolved oxygen, pO₂) und pCO₂ erst möglich. Zusätzlich tragen sowohl das Puffersystem als auch Säure/Base massgeblich zur Osmolalität (Osmo) bei. Das CO₂ unabhängige Puffersystem mit Säure/Base wurde deshalb speziell bezüglich Osmoreduktion entwickelt Die pCO₂-Regelung trägt als zusätzliche Regelgrösse zur Optimierung von Bioprozessen bei und wird eingesetzt zur Steigerung der Prozessperformance durch Opitmierung des pCO₂ Profils, Verbesserung der Reproduzierbarkeit und Verbesserung der Skalierbarkeit.

Ein Vorteil der erfindungsgemäßen Methode ist eine deutliche Verbesserung der spezifischen Produktbildungsrate/ spezifischen Produktivität. Weiterhin kann die Produktqualität, die Fermentationsdauer und der Zellstoffwechsel generell positiv beeinflusst werden. Das heißt beispielsweise, dass die Fermentationsdauer in einem Fed-Batch Verfahren verlängert werden kann bei gleichzeitig höherer Produktausbeute.

Die Implementierung einer weiteren Regelgrösse (pCO₂) ermöglicht eine bessere Prozessoptimierung. Das pH Profil, das Temperatur Profil, das Drehzahl Profil, das O₂ Profil, das pCO₂ Profil können unabhängig voneinander optimiert werden. Die optimierten Bedingungen tragen dann massgeblich zu einer erhöhten Reproduzierbarkeit und Übertragbarkeit auf verschiedenen Bioprozesse und Herstellungsmassstäbe bei. Hierbei ist wichtig, dass nur eine Entkopplung dieser drei benannten Regelgrößen durch Einführung einer separaten pCO₂ Regelung mit neuem Puffersystem eine unabhängige Maßstabsübertragung ermöglicht, in herkömmlichen Systemen beeinflussen sich die 3 Regelgrössen gegenseitig und zwar unterschiedlich stark je nach Maßstab. Deshalb ist es in herkömmlichen Systemen nicht möglich diese 3 Regelgrössen identisch zu übertragen.

Das osmoreduzierte Säure-Base-Puffersystem führt zu einer erhöhten Zellvitalität, steigert die zellspezifische Produktivität und ermöglicht eine weitere Produktivitätssteigerung durch Verlängerung der Prozesszeit. Erfindungsgemäß bevorzugte Säuren und Laugen zur pH-Regulierung sind NaOH (Natronlauge), Phosphorsäure, Schwefelsäure, Salzsäure. Bevorzugt ist Salzsäure.

Ein weiterer Benefit durch das neue, NaHCO₃-freie Medium, ergibt sich im Bereich der Analytik: Bei einigen Versuchen in der Zellkulturtechnik ist es wichtig, die Kohlenstoffdioxid-Bildungsrate (CER) zu bestimmen. Durch Elimination der CO₂ Quellen Puffer, Base und Säure wird eine Kohlenstoffbilanzierung, z.Bsp. über Abgasanalytik überhaupt ermöglicht und zur Prozessoptimierung eingesetzt bzw. einsetzbar.

Die Kohlenstoffbilanzierung erfolgt über die Erfassung, z.B. über Abluftanalytik. Die neue Regelstrategie ermöglicht eine exakte Kohlenstoffbilanzierung (z.Bsp. CER) welche als Optimierungstool eingesetzt wird. Die Kohlenstoffdioxid-Bildungsrate gibt an, welche Menge an CO₂ die Zellen eines Liters Kulturbrühe in einer Stunde bilden. Ist man gezwungen ein Zellkulturmedium zu verwenden, welches über Bicarbonat gepuffert ist, so ergeben sich Eigenheiten in der Bestimmung der Kohlenstoffdioxid-Bildungsrate. Im physiologischen pH-Bereich herrscht beim Gesamtkarbonat ein Gleichgewicht zwischen CO₂ und HCO₃⁻, welches vom pH-Wert beeinflusst wird. Zur Bilanzierung wird die Gaszusammensetzung bei Zugabe und bei Abgabe aus dem Fermenter analysiert. Die Bilanzierung fällt bisher bei NaHCO₃-haltigen Medien schwer, da die eigentliche Speicherwirkung der Zellkulturbrühe unbekannt ist. Das neue, HCO₃⁻ bzw. CO₃-freie Medium, das optimal auf die Zelle abgestimmt ist, eröffnet hier neue Möglichkeiten.

Ein weiterer Benefit der Erfindung ist die Verbesserung der Reproduzierbarkeit von Bioprozessen. Dies bedeutet, dass bei wiederholten Fermentationsläufen in verschiedenen Skalierungen bei kontrolliertem pCO₂ vergleichbare Ergebnisse bezüglich Endtiter, Zellzahl, Metabolismus etc. erzielt werden. Bei Fermentationsläufen, die nicht pCO₂ kontrolliert ablaufen, können diese Parameter von Fermentationslauf zu Fermentationslauf zum Teil erheblich schwanken insbesondere bei unterschiedlicher Skalierung.

### BESCHREIBUNGEN DER ABBILDUNGEN

Der Aufbau des verwendeten Bioreaktors wird anhand Beispiel 3 beispielhaft beschrieben. Der Aufbau des Bioreaktors ist jedoch prinzipiell variabel, um verschiedenen Anforderungen zu entsprechen. Zum Beispiel kann auf ein Steigrohr verzichtet werden und dafür eine weitere Sonde eingebaut oder weitere/andere Lösungen mittels Schlauchpumpen automatisch zugegeben werden.

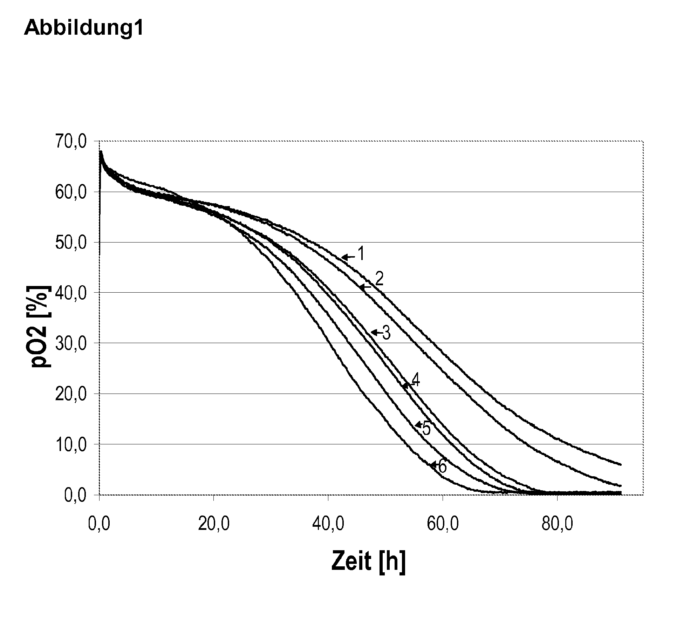

### ABBILDUNG 1 VERGLEICH P02 [%] MIT VARIABLER NAHC03- KONZENTRATION

### Pufferbedingungen:

Kurve 1 = Medium A NaHCO₃-frei + NULL NaHCO₃ (komplett NaHCO₃-frei)
Kurve 2 = Medium A NaHCO₃-frei + 1mM NaHCO₃
Kurve 3 = Medium A NaHCO₃-frei + 4mM NaHCO₃
Kurve 4 = Medium A NaHCO₃-frei + 8mM NaHCO₃ + 20mM HEPES
Kurve 5 = Medium A NaHCO₃-frei + 8mM NaHCO₃
Kurve 6 = Medium A NaHCO₃-frei + 36mM NaHCO₃ (Kontrolle)

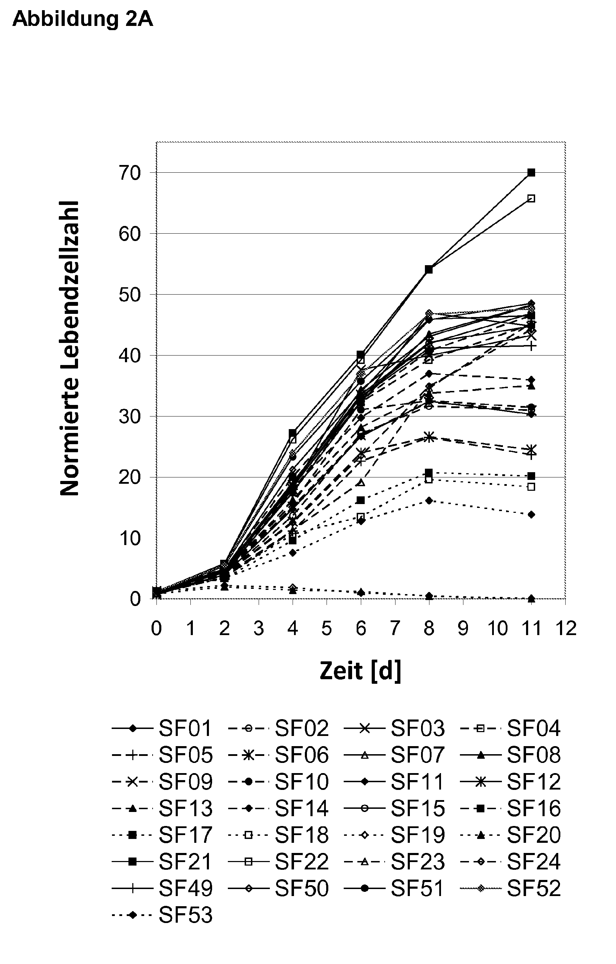

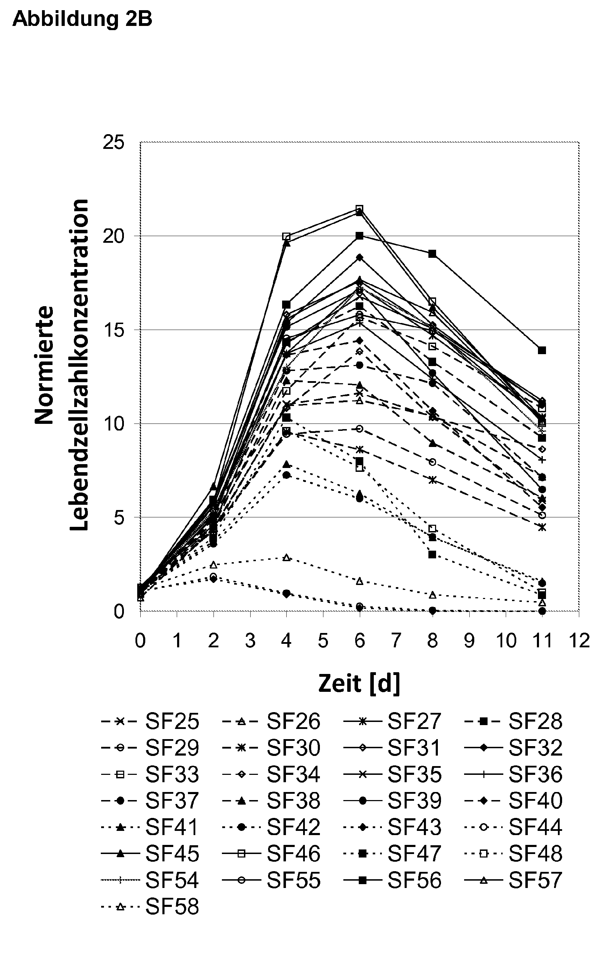

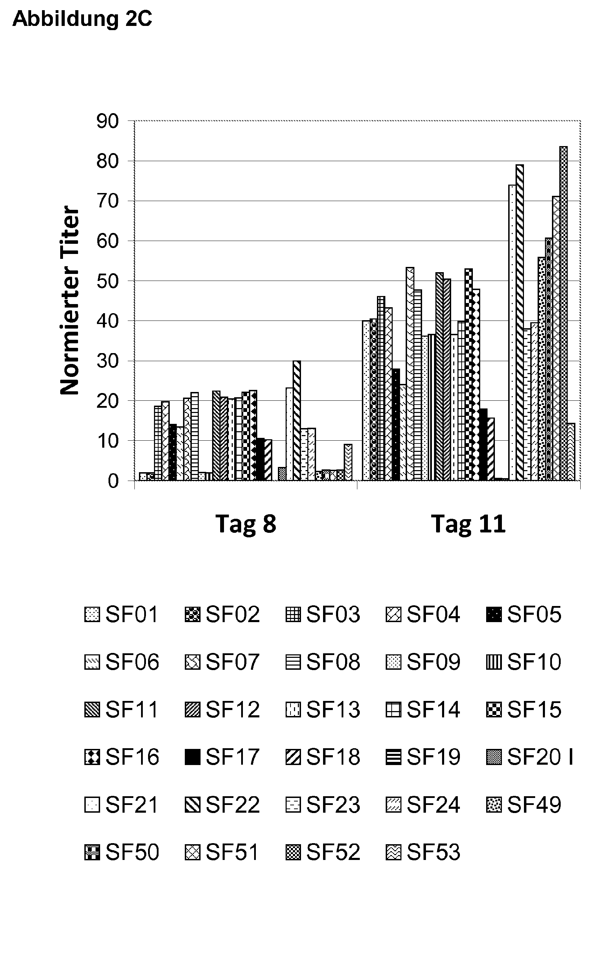

### ABBILDUNG 2 PUFFERSCREEN

### A: Pufferscreen mit hohem pCO2 Profil

SF01 = Medium NaHCO₃-frei mit HEPES-Puffer 100mM (1/2)
SF02 = Medium NaHCO₃-frei mit HEPES-Puffer 100mM (2/2)
SF03 = Medium NaHCO₃-frei mit HEPES-Puffer 60mM (1/2)
SF04 = Medium NaHCO3-frei mit HEPES-Puffer 60mM (2/2)
SF05 = Medium NaHCO₃-frei mit MOPS-Puffer 100mM (1/2)
SF06 = Medium NaHCO₃-frei mit MOPS-Puffer 100mM (2/2)
SF07 = Medium NaHCO₃-frei mit MOPS-Puffer 50mM (1/2)
SF08 = Medium NaHCO₃-frei mit MOPS-Puffer 50mM (2/2)
SF09 = Medium NaHCO₃-frei mit Sod-β 50mM (1/2)
SF10 = Medium NaHCO₃-frei mit Sod-β 50mM (2/2)
SF11 = Medium NaHCO₃-frei mit Sod-β 25mM (1/2)
SF12 = Medium NaHCO₃-frei mit Sod-β 25mM (2/2)
SF13 = Medium NaHCO₃-frei mit TES 80mM (1/2)
SF14 = Medium NaHCO₃-frei mit TES 80mM (2/2)
SF15 = Medium NaHCO₃-frei mit TES 40mM (1/2)
SF16 = Medium NaHCO₃-frei mit TES 40mm (2/2)
SF17 = Medium NaHCO₃-frei mit Trizma-base 50mM (1/2)
SF18 = Medium NaHCO₃-frei mit Trizma-base 50mM (2/2)
SF19 = Medium NaHCO₃-frei mit Trizma-base 100mM (1/2)
SF20 = Medium NaHCO₃-frei mit Trizma-base 100mM (2/2)
SF21 = Medium A (Kontrolle) (1/2)
SF22 = Medium A (Kontrolle) (2/2)
SF23 = Medium A Blank (=Medium A MOPS-frei, NaHCO₃-frei) (1/2)
SF24 = Medium A Blank (=Medium A MOPS-frei, NaHCO₃-frei) (2/2)
SF49 = Medium NaHCO₃-frei mit HEPES-Puffer 60mM + NaHCO₃ 8mM (1/1)
SF50 = Medium NaHCO₃-frei mit MOPS-Puffer 50mM + NaHCO₃ 8mM (1/1)
SF51 = Medium NaHCO₃-frei mit Sod-β 25mM + NaHCO₃ 8mM (1/1)
SF52 = Medium NaHCO₃-frei mit TES-Puffer 40mM + NaHCO₃ 8mM (1/1)
SF53 = Medium NaHCO₃-frei mit Trizma-base-Puffer 50mM + NaHCO₃ 8mM (1/1)

### B: Pufferscreen mit niedrigem pCO2 Profil

SF25 = Medium NaHCO₃-frei mit HEPES-Puffer 100mM (1/2)
SF26 = Medium NaHCO₃-frei mit HEPES-Puffer 100mM (2/2)
SF27 = Medium NaHCO₃-frei mit HEPES-Puffer 60mM (1/2)
SF28 = Medium NaHCO₃-frei mit HEPES-Puffer 60mM (2/2)
SF29 = Medium NaHCO₃-frei mit MOPS-Puffer 100mM (1/2)
SF30 = Medium NaHCO₃-frei mit MOPS-Puffer 100mM (2/2)
SF₃1 = Medium NaHCO₃-frei mit MOPS-Puffer 50mM (1/2)
SF32 = Medium NaHCO₃-frei mit MOPS-Puffer 50mM (2/2)
SF33 = Medium NaHCO₃-frei mit Sod-β 50mM (1/2)
SF34 = Medium NaHCO₃-frei mit Sod-β 50mM (2/2)
SF35 = Medium NaHCO₃-frei mit Sod-β 25mM (1/2)
SF36 = Medium NaHCO₃-frei mit Sod-β 25mM (2/2)
SF37 = Medium NaHCO₃-frei mit TES 80mM (1/2)
SF38 = Medium NaHCO₃-frei mit TES 80mM (2/2)
SF39 = Medium NaHCO₃-frei mit TES 40mM (1/2)
SF40 = Medium NaHCO₃-frei mit TES 40mM (2/2)
SF41 = Medium NaHCO₃-frei mit Trizma-base 50mM (1/2)
SF42 = Medium NaHCO₃-frei mit Trizma-base 50mM (2/2)
SF43 = Medium NaHCO₃-frei mit Trizma-base 100mM (1/2)
SF44 = Medium NaHCO₃-frei mit Trizma-base 100mM (2/2)
SF45 = Medium A (Kontrolle) (1/2)
SF46 = Medium A (Kontrolle) (2/2)
SF47 = Medium A Blank (=Medium A MOPS-frei, NaHCO₃-frei) (1/2)
SF48 = Medium A Blank (=Medium A MOPS-frei, NaHCO₃-frei) (2/2)
SF54 = Medium NaHCO₃-frei mit HEPES-Puffer 60mM + NaHCO₃ 8mM (1/1)
SF55 = Medium NaHCO₃-frei mit MOPS-Puffer 50mM + NaHCO₃ 8mM (1/1)
SF56 = Medium NaHCO₃-frei mit Sod-β 25mM + NaHCO₃ 8mM (1/1)
SF57 = Medium NaHCO₃-frei mit TES-Puffer 40mM + NaHCO₃ 8mM (1/1)
SF58 = Medium NaHCO₃-frei mit Trizma-base-Puffer 50mM + NaHCO₃ 8mM (1/1)

### C: Pufferscreen bezüglich Titer an Tag 8 und 11

SF01 = Medium NaHCO₃-frei mit HEPES-Puffer 100mM (1/2)
SF02 = Medium NaHCO₃-frei mit HEPES-Puffer 100mM (2/2)
SF03 = Medium NaHCO₃-frei mit HEPES-Puffer 60mM (1/2)
SF04 = Medium NaHCO₃-frei mit HEPES-Puffer 60mM (2/2)
SF05 = Medium NaHCO₃-frei mit MOPS-Puffer 100mM (1/2)
SF06 = Medium NaHCO₃-frei mit MOPS-Puffer 100mM (2/2)
SF07 = Medium NaHCO₃-frei mit MOPS-Puffer 50mM (1/2)
SF08 = Medium NaHCO₃-frei mit MOPS-Puffer 50mM (2/2)
SF09 = Medium NaHCO₃-frei mit Sod-β 50mM (1/2)
SF10 = Medium NaHCO₃-frei mit Sod-β 50mM (2/2)
SF11 = Medium NaHCO₃-frei mit Sod-β 25mM (1/2)
SF12 = Medium NaHCO₃-frei mit Sod-β 25mM (2/2)
SF13 = Medium NaHCO₃-frei mit TES 80mM (1/2)
SF14 = Medium NaHCO₃-frei mit TES 80mM (2/2)
SF15 = Medium NaHCO₃-frei mit TES 40mM (1/2)
SF16 = Medium NaHCO₃-frei mit TES 40mM (2/2)
SF17 = Medium NaHCO₃-frei mit Trizma-base 50mM (1/2)
SF18 = Medium NaHCO₃-frei mit Trizma-base 50mM (2/2)
SF19 = Medium NaHCO₃-frei mit Trizma-base 100mM (1/2)
SF20 = Medium NaHCO₃-frei mit Trizma-base 100mM (2/2)
SF21 = Medium A (Kontrolle) (1/2)
SF22 = Medium A (Kontrolle) (2/2)
SF23 = Medium A Blank (=Medium A MOPS-frei NaHCO₃-frei) (1/2)
SF24 = Medium A Blank (=Medium A MOPS-frei NaHCO₃-frei) (2/2)
SF49 = Medium NaHCO₃-frei mit HEPES-Puffer 60mM + NaHCO₃ 8mM (1/1)
SF50 = Medium NaHCO₃-frei mit MOPS-Puffer 50mM + NaHCO₃ 8mM (1/1)
SF51 = Medium NaHCO₃-frei mit Sod-β 25mM + NaHCO₃ 8mM (1/1)
SF52 = Medium NaHCO₃-frei mit TES-Puffer 40mM + NaHCO₃ 8mM (1/1)
SF53 = Medium NaHCO₃-frei mit Trizma-base-Puffer 50mM + NaHCO₃ 8mM (1/1)

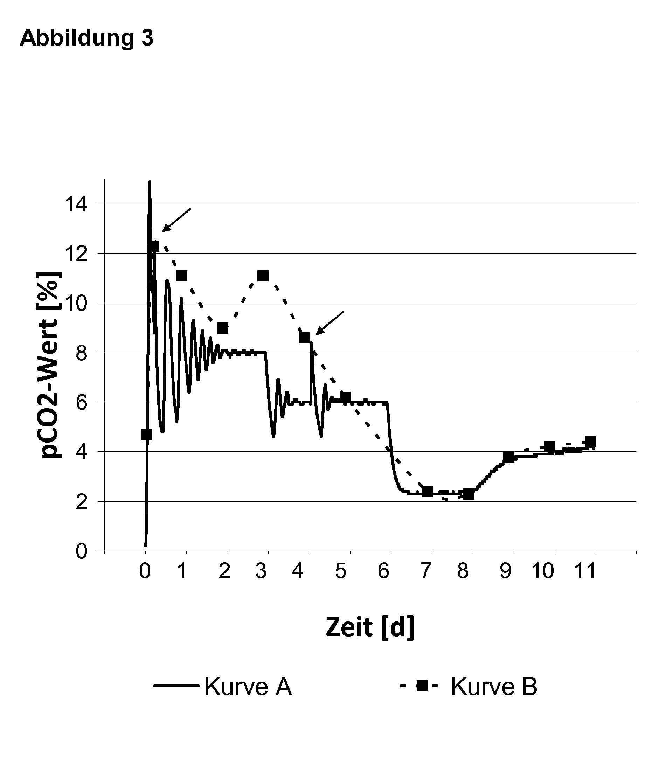

### ABBILDUNG 3 PCO₂ REGELUNG

Die Abbildung 3 zeigt grafisch den Verlauf der internen pCO₂ Messung (Sonde mit automatischer Datenarchivierung) und des externen Messwertes am Blutgasanalyseautomat (BGA) aufgetragen über die Zeit. Die mit einem Pfeil markierte erste Kalibration (Abgleich zwischen CO₂ Sonde und BGA) an Tag 0 (d0) zeigt wenig Effekt, da das Regelverhalten noch zu unruhig ist. Ab der 2. Kalibration (2. Pfeil) an Tag 4 (d4) ist eine gute Übereinstimmung zwischen internem und externem Messwert zu erkennen. Achsendarstellung: pCO₂-online vs pCO₂-BGA
Kurve A = CO₂-Messwert intern (via CO₂-Sonde, Fa. Mettler Toledo)
Kurve B = CO₂-Messwert extern (via BGA Rapidlap, Fa. Siemens)

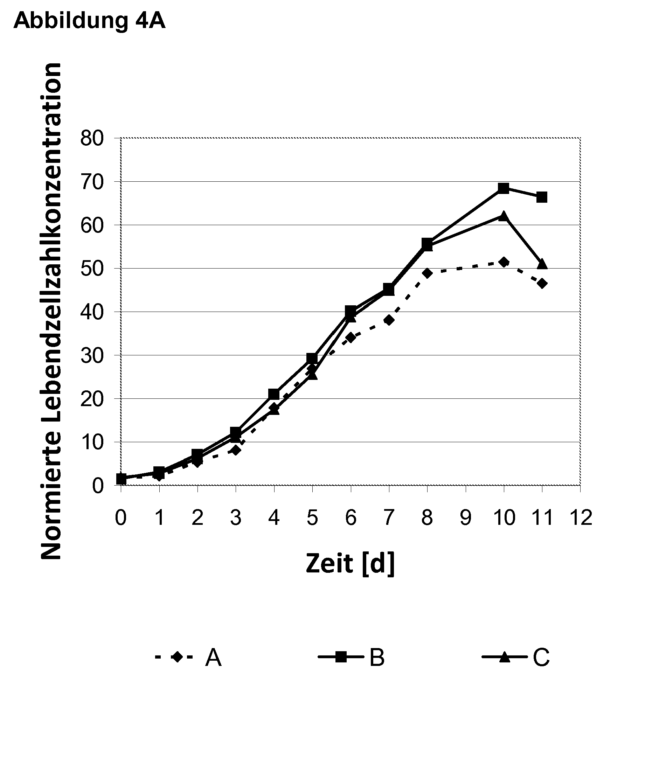

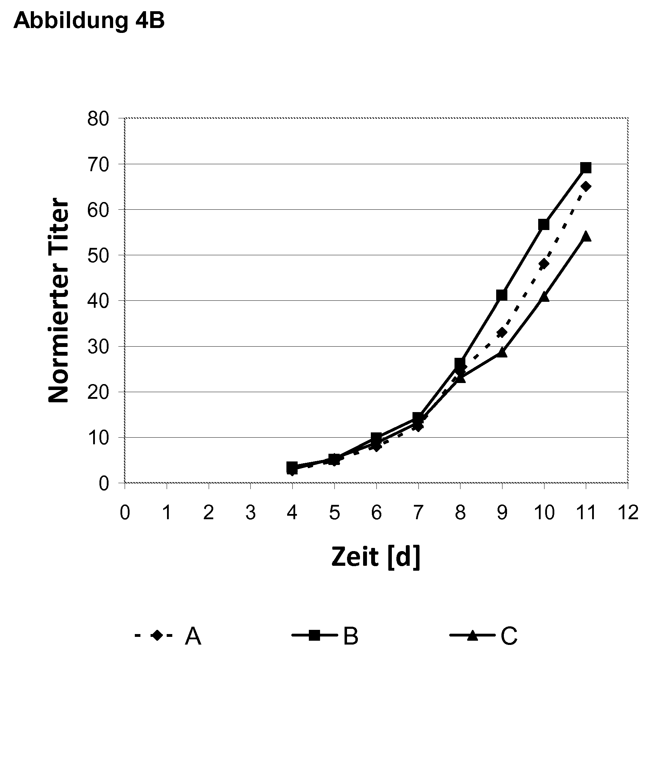

### ABBILDUNG 4 NORMALE LEBENDZELLZAHLKONZENTRATION (A) BZW. TITERKONZENTRATION (B)

A: Lebendzellzahlkonzentration (Vgl. pCO₂-Geregelt/Ungeregelt)
A = FS 33.1 Medium B NaHCO₃⁻-frei MOPS-frei + 40mM TES (pCO₂-geregelt) pH-Agens: NaOH
B = FS 32.2 Medium B NaHCO₃-frei MOPS-frei + 40mM TES+ 8mM NaHCO₃ (ungeregelt) pH-Agens: NaOH
C = FS 32.3 Medium B , NaHCO₃-frei , MOPS-frei + 40mM TES + 8mM NaHCO₃ (ungeregelt) pH-Agens: Na₂CO₃
B: Normierter Titer (Vgl. pCO₂-Geregelt/Ungeregelt)
A = FS 33.1 Medium B , NaHCO₃-frei , MOPS-frei + 40mM TES (pCO₂-geregelt) pH-Agens: NaOH
B = FS 32.2 Medium B , NaHCO₃-frei , MOPS-frei + 40mM TES + 8mM NaHCO₃ (ungeregelt) pH-Agens: NaOH
C = FS 32.3 Medium B , NaHCO₃-frei , MOPS-frei + 40mM TES+ 8mM NaHCO₃ (ungeregelt) pH-Agens: Na₂CO₃

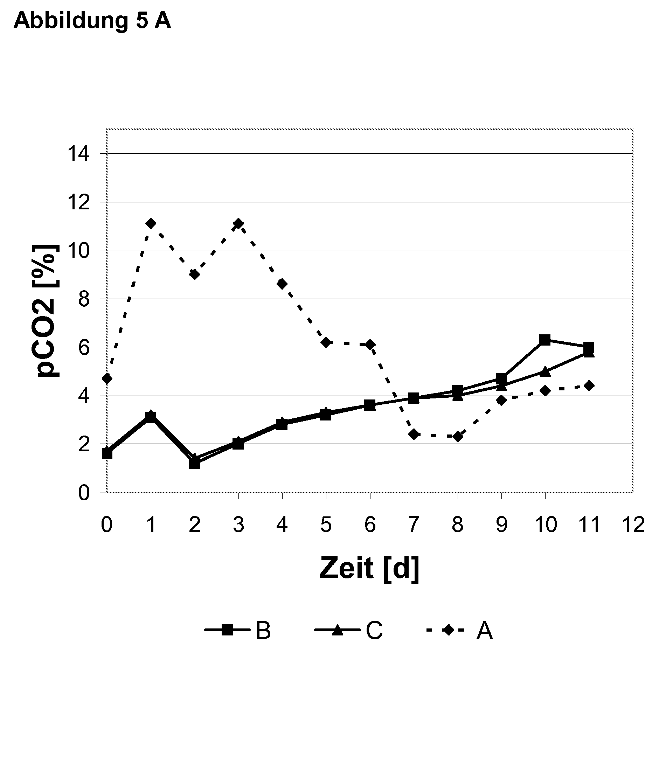

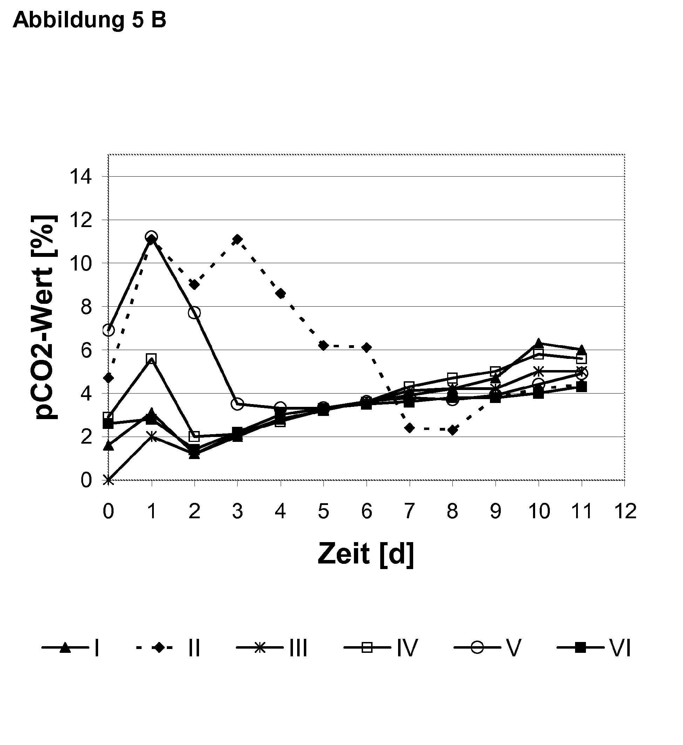

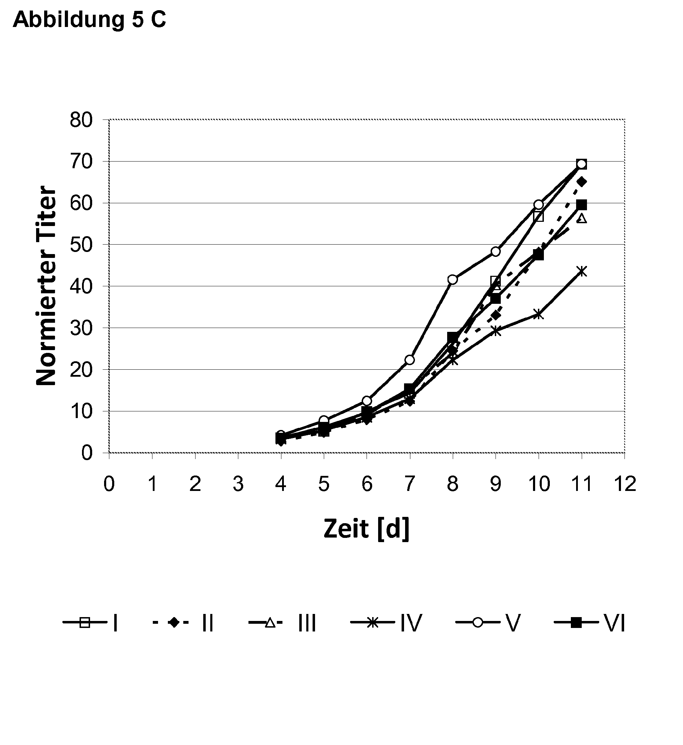

### ABBILDUNG 5 VERGLEICH PUFFERSYSTEME

### A: pCO₂-Profil geregelt/ungeregelt

A = FS 33.1 Medium B , NaHCO₃-frei , MOPS-frei + 40mM TES (pC02-geregelt) pH-Agens: NaOH
B = FS 32.2 Medium B , NaHCO₃-frei , MOPS-frei + 40mM TES + 8mM NaHCO₃ (ungeregelt) pH-Agens: NaOH
C = FS 32.3 Medium B , NaHCO₃-frei , MOPS-frei + 40mM TES + 8mM NaHCO₃ (ungeregelt) pH-Agens: Na₂CO₃

### B: pCO2-Profil geregelt(1x) /ungeregelt(5x)

1 = FS 32.2 Medium B , NaHCO₃-frei , MOPS-frei + TES 40mM + NaHCO₃ 8mM (NaOH)
II = FS 33.1 Medium B , NaHCO₃-frei , MOPS-frei + TES 40mM (pCO2-geregelt) (NaOH)
III = FS 32.4 Medium B , NaHCO₃-frei , MOPS-frei + Sod-β 25mM (NaOH)
IV = FS 32.5 Medium B , NaHCO₃-frei , MOPS-frei + Sod-ß 25mM + NaHCO₃ 12mM (NaOH)
V = FS 33.3 Medium B , NaHCO₃-frei , MOPS-frei + NaHCO₃ 36mM (Vergl.-Kontrolle) (NaOH)
VI = FS 33.5 Medium B , NaHCO₃-frei , MOPS-frei + NaHCO₃ 8mM (NaOH)

### C: Normierter Titer

I = FS 32.2 Medium B , NaHCO₃-frei , MOPS-frei + TES 40mM + NaHCO₃ 8mM (NaOH)
II = FS 33.1 Medium B , NaHCO₃-frei , MOPS-frei + TES 40mM (pCO2-geregelt) (NaOH)
III = FS 32.4 Medium B , NaHCO₃-frei , MOPS-frei + Sod-ß 25mM (NaOH)
IV = FS 32.5 Medium B , NaHCO₃-frei , MOPS-frei + Sod-β 25mM + NaHCO₃ 12mM (NaOH)
V = FS 33.3 Medium B , NaHCO₃-frei , MOPS-frei + NaHCO₃ 36mM (Vergl.-Kontrolle) (NaOH)
VI = FS 33.5 Medium B , NaHCO₃-frei , MOPS-frei + NaHCO₃ 8mM (NaOH)

Die mit TES Puffer erreichten Produkttiter bzw. Produktkonzentrationen sind auf gleichem Niveau wie diejenigen der Kontrolle.

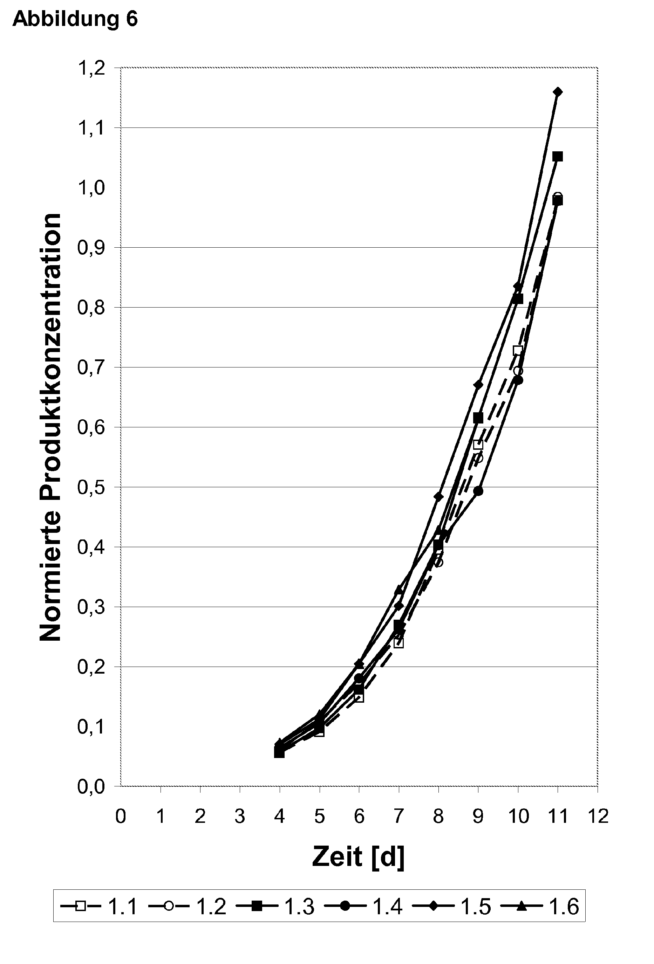

### ABBILDUNG 6 VERGLEICH PUFFERSYSTEME NORMIERTE PRODUKTKONZENTRATION

Läufe 1.1 bis 1.6 in Abbildung 6 mit CO₂-Regelung und NaOH 1,2M als Lauge.
1.1 = FS 33.1 Medium B, NaHCO₃-frei, MOPS-frei + TES 40mM
1.2 = FS 33.2 Medium B, NaHCO₃-frei, MOPS-frei + TES 40mM + NaHCO₃ 8mM
1.3 = FS 33.2 Medium B, NaHCO₃-frei, MOPS-frei + Sod-β 25mM
1.4 = FS 33.2 Medium B, NaHCO₃-frei, MOPS-frei + Sod-β 25mM + NaHCO₃ 8mM
1.5 = FS 33.2 Medium B, NaHCO₃-frei, MOPS-frei + Sod-β 25mM + NaHCO₃ 12mM
1.6 = FS 33.2 Medium B, NaHCO₃-frei, MOPS-frei + Sod-β 25mM + NaHCO₃ 16mM

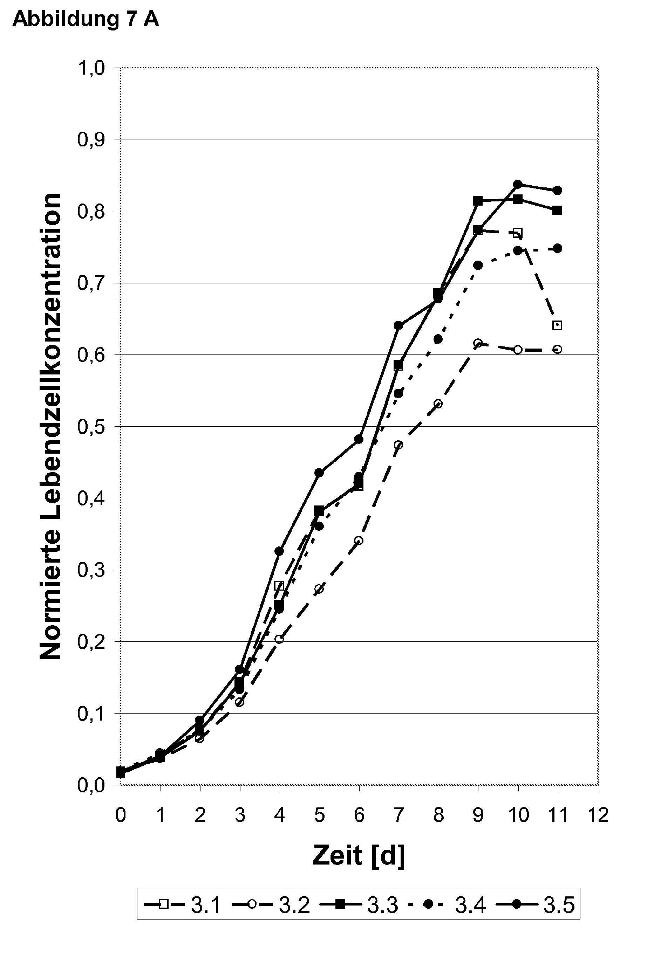

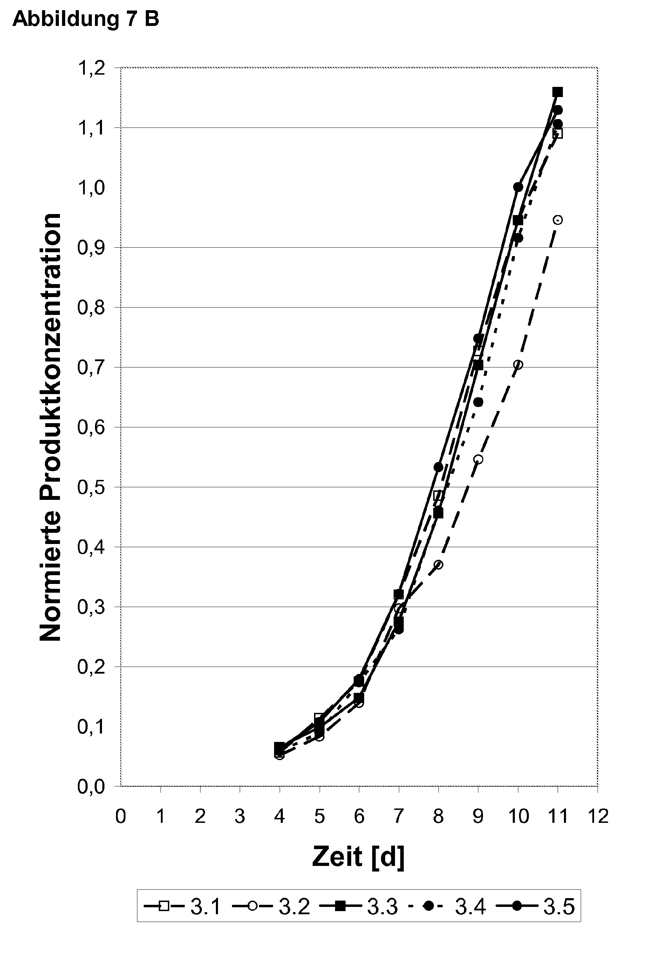

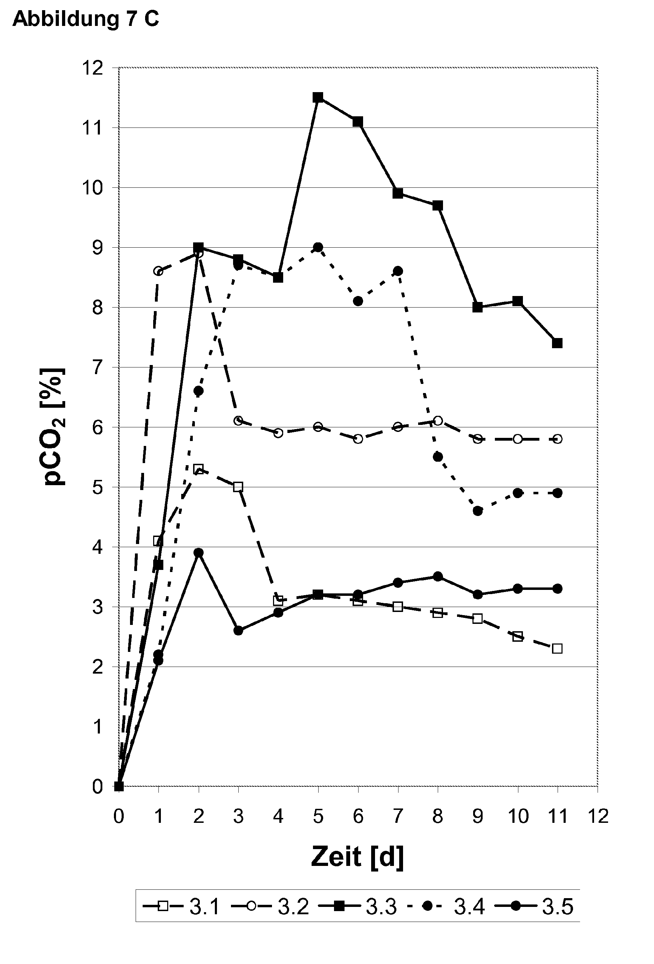

### ABBILDUNG 7 VERGLEICH CO₂-PROFILE

Läufe 3.1 bis 3.5 in Abbildung 7 mit CO₂-Regelung, NaOH 1,2M als Lauge und mit Medium B, NaHCO₃-frei, MOPS-frei + Sod-β 25mM

### Verschiedene CO₂-Profile:

3.1 = Tag 0-3 5% CO₂, Tag 3-11 3% CO₂
3.2 = Tag 0-2 8% CO₂, Tag 2-11 6% CO₂
3.3 = Tag 0-4 8% CO₂, Tag 4-8 10% CO₂, Tag 8-11 8% CO₂
3.4 = Tag 0-1 8% CO₂, Tag 1-2 6% CO₂, Tag 2-7 8% CO₂, Tag 7-11 5% CO₂
3.5 = Tag 0-11 3% CO₂

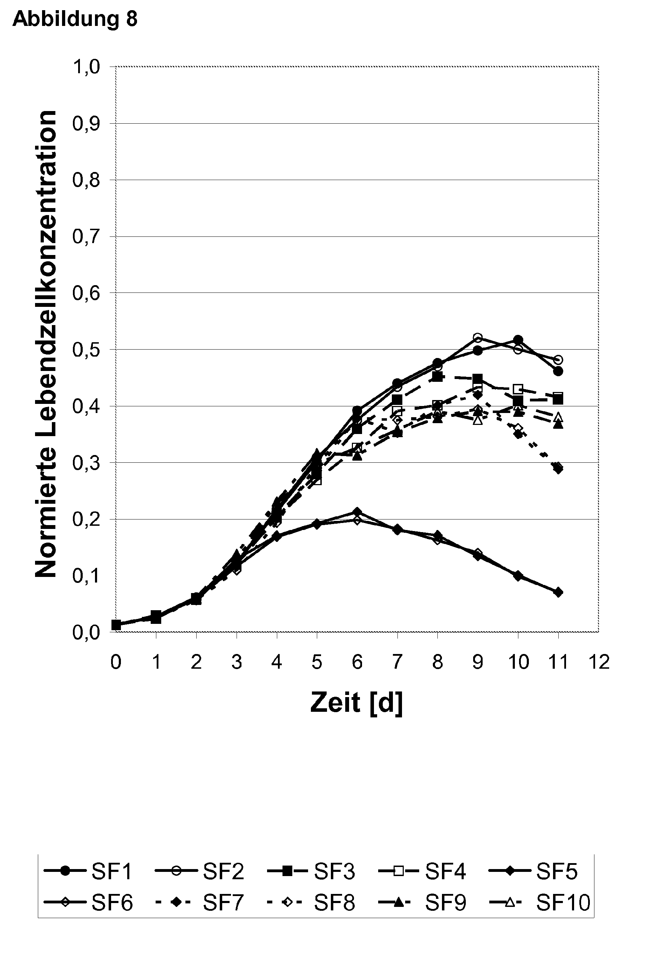

### ABBILDUNG 8 SÄUREVERTRÄGLICHKEIT MIT EINSATZ DER NA-SALZE

SF1-10 mit Einsatz des Medium B, NaHCO₃-frei, MOPS-frei + Sod-ß 25mM und Zugabe verschiedener Na-Salze an Tag 3:
SF1 = Zugabe von Wasser (für Kontrolle)
SF2 = Zugabe von Wasser (für Kontrolle)
SF3 = Zugabe von Natriumchlorid (für Salzsäure)
SF4 = Zugabe von Natriumchlorid (für Salzsäure)
SF5 = Zugabe von Natriumacetat (für Essigsäure)
SF6 = Zugabe von Natriumacetat (für Essigsäure)
SF7 = Zugabe von Natriumsulfat (für Schwefelsäure)
SF8 = Zugabe von Natriumsulfat (für Schwefelsäure)
SF9 = Zugabe von Di-Natriumhydrogenphosphat (für Phosphorsäure)
SF10 = Zugabe von Di-Natriumhydrogenphosphat (für Phosphorsäure)

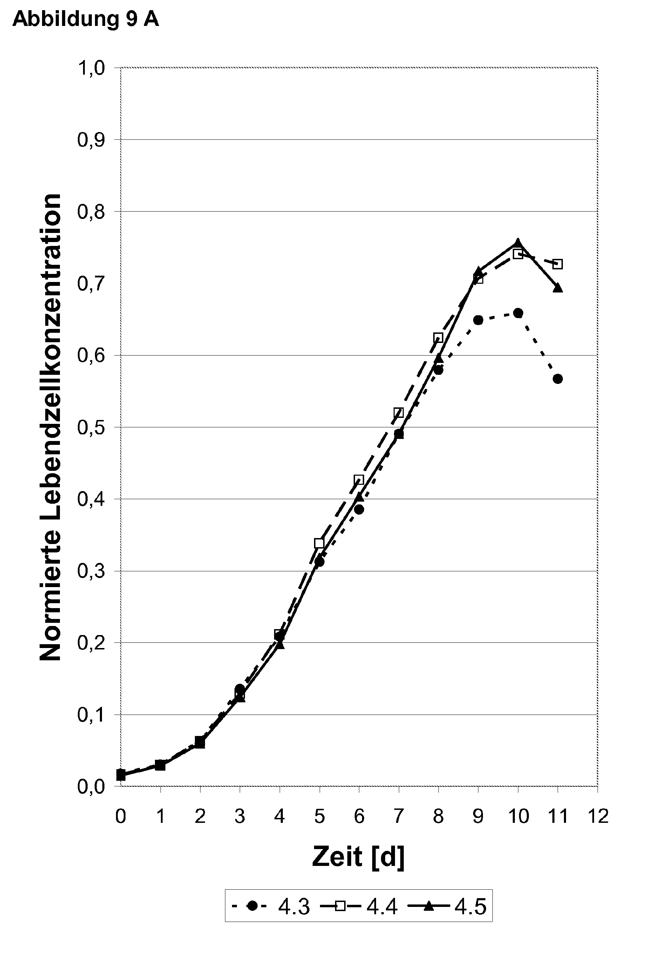

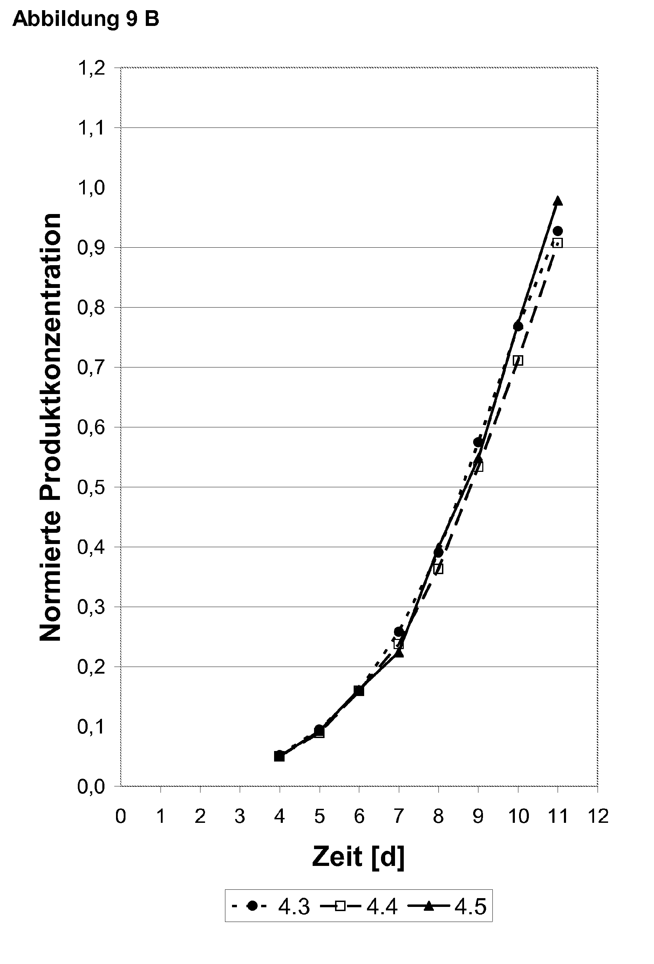

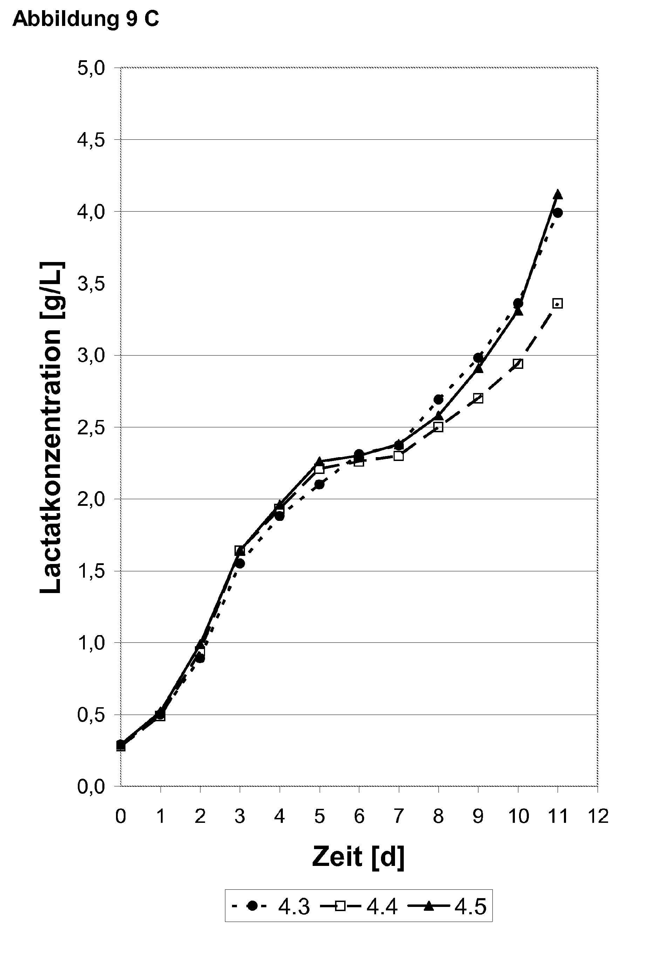

### ABBILDUNG 9 VERGLEICH VERSCHIEDENER SÄUREN

Läufe 4.3 bis 4.5 in Abbildung 9 mit CO₂-Regelung, NaOH 1,2M als Lauge, mit Medium B, NaHCO₃-frei, MOPS-frei + Sod-ß 25mM und Zugabe verschiedener Säuren (50 mL) an Tag 3:
4.3 = Zugabe von Salzsäure 99mM
4.4 = Zugabe von Phosphorsäure 81mM
4.5 = Zugabe von Schwefelsäure 54mM

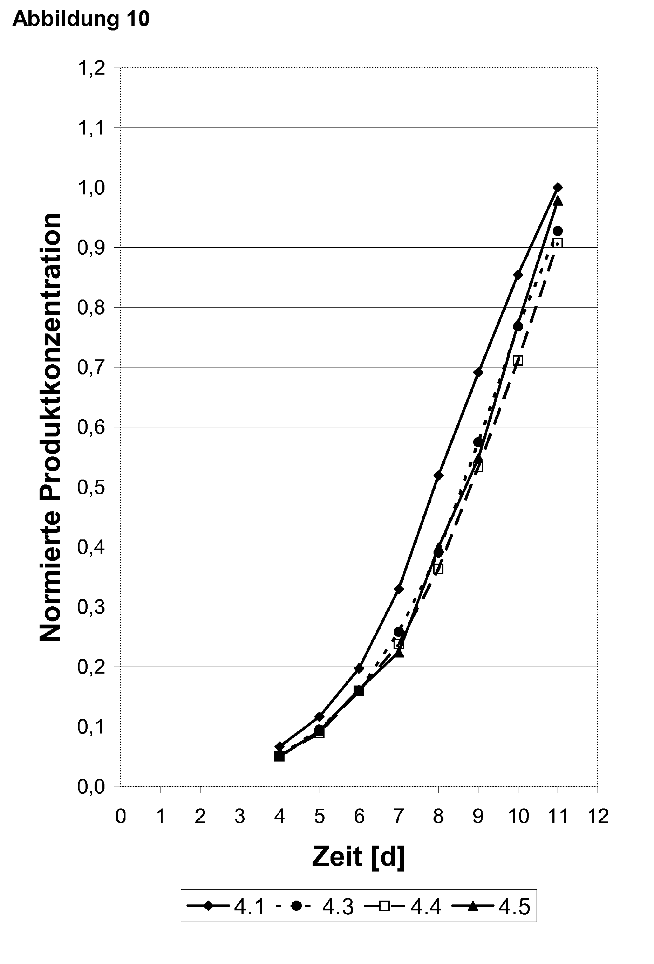

### ABBILDUNG 10 VERGLEICH STANDARDPROZESS OHNE CO₂-REGELUNG MIT CO₂-GEREGELTEM PROZESS

4.1 = Medium B, Na₂CO₃ 1M als Lauge, ohne CO₂-Regelung (Standardprozess)
4.3 = Medium B, NaHCO₃-frei, MOPS-frei + Sod-ß 25mM, NaOH 1,2M als Lauge, mit CO₂-Regelung und Zugabe von Salzsäure 99mM
4.4 = Medium B, NaHCO₃-frei, MOPS-frei + Sod-ß 25mM, NaOH 1,2M als Lauge, mit CO₂-Regelung und Zugabe von Phosphorsäure 81mM
4.5 = Medium B, NaHCO₃-frei, MOPS-frei + Sod-ß 25mM, NaOH 1,2M als Lauge, mit CO₂-Regelung und Zugabe von Schwefelsäure 54mM

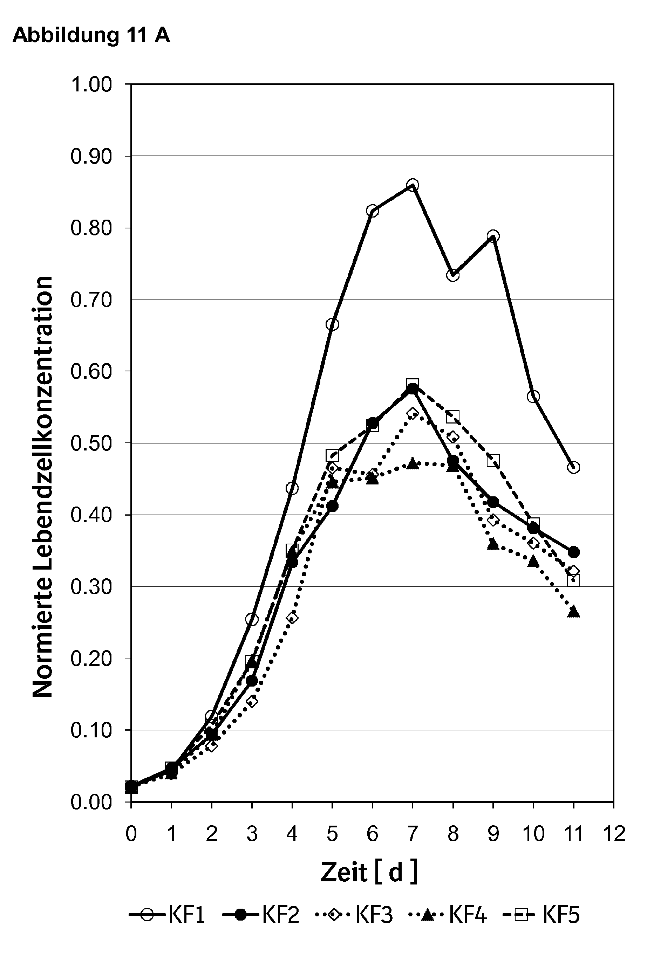

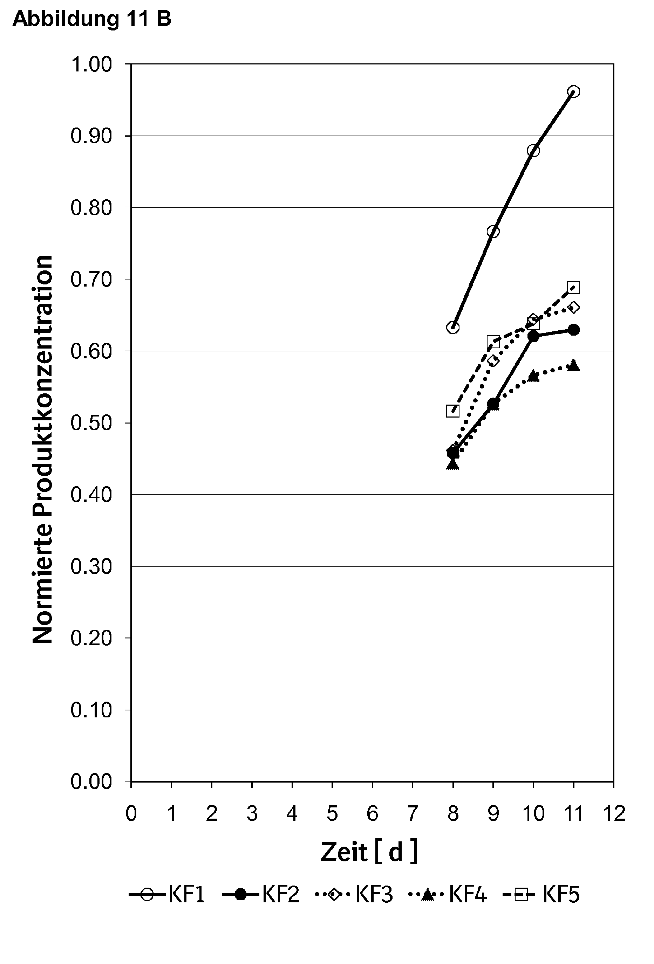

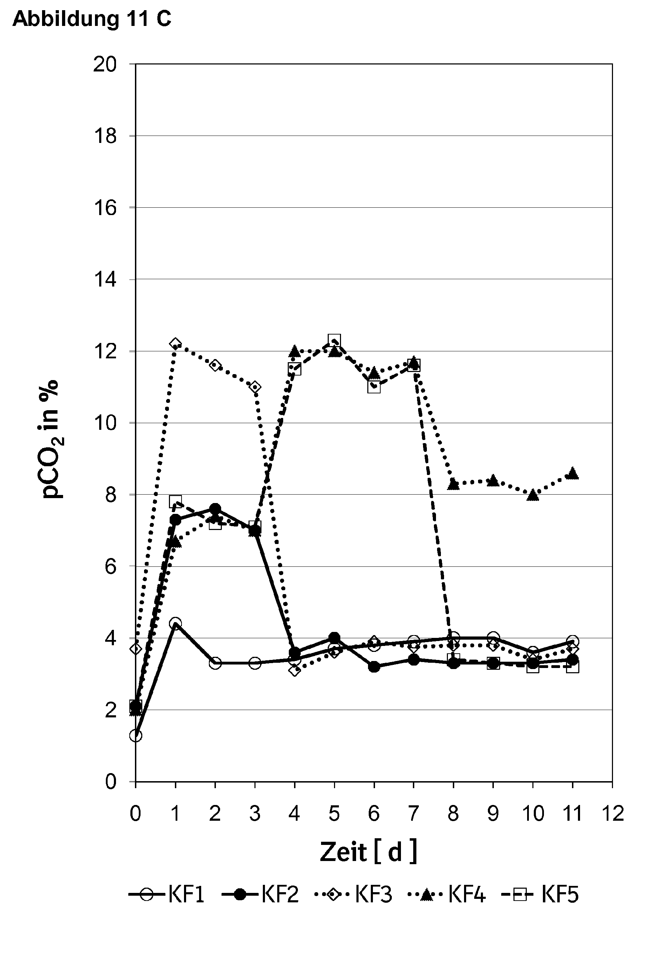

### ABBILDUNG 11 VERGLEICH CO₂-PROFILE MIT MODELLZELLE 2

### (A)Normierte Zellkonzentration, (B) Normierte Produktkonzentration, (C) CO₂ Profile Läufe KF1 bis KF5 in Abbildung 11 mit CO₂-Regelung, NaOH 1,2M als Lauge und mit Medium B, NaHCO₃-frei, MOPS-frei + Sod-ß 25mM

### Verschiedene CO₂-Profile:

KF1 = Tag 0-11 3% CO₂
KF2 = Tag 0-3 7% CO₂, Tag 3-11 3% CO₂
KF3 = Tag 0-3 11% CO₂, Tag 3-11 3% CO₂
KF4 = Tag 0-3 7% CO₂, Tag 3-7 11% CO₂, Tag 7-11 7% CO₂
KF5 = Tag 0-3 7% CO₂, Tag 3-7 11% CO₂, Tag 7-11 3% CO₂

### DETAILLIERTE BESCHREIBUNG DER ERFINDUNG

Die Experimente zeigen beispielhaft:

### Resultat 1: Die Reduzierte NaHC03 Konzentration von 12mmol/L und speziell von 8mmol/L zeigt ein vorteilhaftes, der Kontrolle ähnliches pO₂ Profil (Zellwachstum).

### Resultat 2:

- > Identifizierung der besten Puffersubstanzen bei NaHCO₃ freiem Zellkulturmedium bzw. bei NaHCO₃ erniedrigtem (=8mmol/L) Zellkulturmedium.
- > Die besten Puffersubstanzen sind: TES (z.B. 40mmol/L) und Sod-ß (z.B. 25mmol/L)

In Abbildung 2A ist ersichtlich, dass alle Puffermedienansätze bis auf Trizma-base (SF19 und SF20) mit Konzentration I Zellwachstum zeigen. Das beste Wachstum zeigen die beiden Kontrollen (SF21 und SF22), doch ist der Unterschied zu den Puffermedienansätzen, zumindest bis d8, relativ klein. Die drei besten Puffer, rangierend bei einer Zelldichte um 45, sind Sod-ß / H; TES / K + NaHCO₃ / D und MOPS / F + NaHCO₃ / D. Trizma-base läuft in jeglicher Konzentration schlecht, der pH ist zwar stabil aber evtl. liegt eine Toxizität vor.

In Abbildung 2B ist der Maßstab von Abbildung 2A beibehalten worden. Dabei sieht man deutlich, dass die erreichten Zelldichten bei diesem CO₂-Profil wesentlich geringer ausfallen. Die Kontrollen schneiden zwar auch hier am Besten ab, doch ist eine maximale Zelldichte von 20 nicht akzeptabel. Die zwei besten Pufferansätze, rangierend bei einer Zelldichte um 18, sind hier Sod-ß / H + NaHCO₃ / D und MOPS / F. Aus diesem Grund beschränkt sich die weitere Auswertung primär auf die Shakeflasks aus Brutschrank 1 mit dem dazugehörigen CO₂-Profil.

Generell ist die Kombination mit NaHCO₃ / D bei den niedrigen Pufferkonzentrationen vorteilhaft.

In Abbildung 2C sind die erreichten Titerkonzentrationen (normiert) aufgetragen. Bis auf wenige Ausnahmen ist ein ansteigender Titer zwischen d8 und d11 erkennbar. Der Zugewinn ist dabei bei den Shakeflasks SF49 bis SF52, den Medien mit der reduzierten NaHCO₃-Konzentration, sogar mehr als zehnfach!

Bemerkenswert ist auch die Tatsache, dass erstmals mit einer neuen Puffersubstanz im Medium die herkömmliche Kontrolle (SF21 u. SF22) überholt wurde: SF52 mit TES / K + NaHCO₃ / D liefern am d11 mit norm. Produktkonzentration 83,6 ein sehr gutes Ergebnis.

### Resultat 3

Aus den pCO₂ Regelungsexperimenten ist ersichtlich, dass mit einem pCO₂-Sollwert von 2% eine kritische Untergrenze erreicht wird, welche im Prozess nicht sinnvoll erscheint.

### Resultat 4:

Abbildung 4A und B zur normalen Lebendzellzahlkonzentration (A) bzw. Titerkonzentration (B) zeigt, dass die zellspezifische Produktbildungsrate des geregelten Fermentationslaufes (Kurve mit Dreiecksymbol) höher ist als diejenige des vergleichbaren ungeregelten Fermentationslaufes (Kurve mit Raute). Weiterhin zeigen die Versuche aus Abbildung 4, dass die pH-Regelung mit NaOH tendenziell eine bessere Produktbildungsrate aufweist als diejenige mit Na₂CO₃. Bei einer weiteren Optimierung des pCO₂ Profils wird die spezifische Produktbildungsrate weiter erhöht. Die Lebendzellzahldichte des pCO₂-geregelten Laufs (FS 33.1) weicht, wie in Abbildung 4B ersichtlich um maximal 20 Einheiten voneinander ab. Ab d10 streuen auch die beiden Vergleichsläufe gering, sonst wird eine gute Übereinstimmung erzielt. Das niedrigere Niveau von FS 33.1 kann aus dem höheren pCO₂-Profil resultieren und ist nicht negativ zu interpretieren. Die Vitalität (nicht gezeigt) ist bei allen Läufen auf gleichem Niveau. Die Laugenverbräuche (nicht gezeigt) sind bei allen Läufen gering. Bei FS 32.2 (höchste Lebendzellzahlkonzentration) muss gegen Ende der Fermentation (d9-d11) erwartungsgemäß mehr Lauge zugegeben werden. Trotzdem weißt dieser Lauf am Fermentationsende mit 393 mosmol/kg die geringste Osmolalität auf (nicht gezeigt). In Abbildung 4A ist ersichtlich, dass FS 33.1 trotz geringerer Zellzahl als FS 32.3 eine höhere Titerkonzentration aufweißt. Die Konzentration liegt im oberen Drittel der Streuung zwischen den beiden identischen Fermentationsläufen FS 32.2 und FS 32.3. Das heißt, dass der Fermentationslauf mit der pCO₂-Regelung eine höhere spezifische Produktbildungsrate besitzt.

### Resultat 5:

-> Optimalerweise läuft ein Fermentationslauf unter folgenden Bedingungen ab: TES-Puffer (bzw. Sod-ß Puffer) komplett ohne NaHCO₃ und mit pCO₂ Regelung. Hiermit werden die besten Endtiter erreicht.

Abbildung 5A zeigt, dass sich das pCO₂ Profil des geregelten Fermentationslaufes (FS33.1) deutlich von den Vergleichsfermentationen (FS32.2 und FS32.3) abhebt. FS32.2 und FS32.3 werden zum Vergleich herangezogen obwohl sie einen geringen Anteil von NaHC03 aufweisen, da aufgrund einer Kontamination ein identischer Vergleichspartner entfallen ist. Das durch die Regelung erzielte pCO₂ Profil hebt sich im ersten Drittel der Fermentation deutlich von den Vergleichsläufen ab und folgt der Sollvorgabe (siehe Abbildung 5B, d1-d3 pCO₂ = 8%, d3-d6 pCO₂ = 6%, d6-d8 pCO₂ = 2% und von d8-d11 pCO₂ = 4%) weitestgehend. Beim Erzwingen eines niedriger als normalen pCO₂ Wertes (Tag 6-8, d6-d8) wird Stickstoff eingegast um CO₂ auszutreiben. Es ist festzustellen, dass der niedrige Sollwert nie erreicht wird und ein pCO₂ Wert von 3.2% eine kritische Untergrenze darstellt für die Regelung. Bei der dabei erhöhten Anforderung von Stickstoff wird im Zusammenhang auch Sauerstoff ausgetrieben, welcher durch einen höheren Sauerstoffvolumenstrom ausgeglichen wird. Es kann einer kritischen Gesamtgasmenge erreicht werden, bei welcher eine unverhältnismäßig große Schaumbildung festzustellen ist.

Aus Abbildung 5B ist ersichtlich, dass sich der geregelte Fermentationslauf auch von allen anderen Vergleichsläufen überraschend deutlich abhebt. Das heißt, dass das durch die Regelung eingestellte pCO₂ Profil tatsächlich auf der Sollvorgabe (d1-d3 pCO₂ = 8%, d3-d6 pCO₂ = 6%, d6-d8 pCO₂ = 2% und von d8-d11 pCO₂ = 4%) beruht und nicht zufällig zustande kommt.

Abbildung 5C zeigt, dass die mit TES ohne NaHCO₃ bzw. TES mit 8mmol/L NaHCO₃ erreichten Titerkonzentrationen vergleichbar sind mit der Kontrolle (NaHCO₃ basierter Puffer mit einer Konzentration von 36mmol/L). Der Lauf mit TES ohne NaHCO₃ (geregelt) erreicht trotz zufällig gewählten und nicht optimierten pCO₂ Profil eine Endkonzentration (Titer), welche sich um lediglich 6.10% (gemessen an der Kontrolle) von der Kontrolle unterscheidet. Der Lauf mit TES plus 8mmol/L NaHCO₃ (ungeregelt) erreicht eine Endkonzentration (Titer), welche sich um lediglich 0.01% (gemessen an der Kontrolle) von der Kontrolle unterscheidet. Dies zeigt vollkommen überraschend, dass NaHCO₃ vollständig durch TES ersetzt werden kann. Weiterhin zeigt dies, dass das Ergebnis des Laufs mit TES plus 8mmol/L NaHCO₃ (ungeregelt) die gleiche Endtiterkonzentration erreicht wie die Kontrolle.

### Resultat 6:

Für das Erreichen einer möglichst hohen Produktkonzentration ist der Einsatz von NaHCO₃ im Medium überraschenderweise nicht erforderlich. Die in Abbildung 6 dargestellten Produktkonzentrationen beider Läufe mit Einsatz von TES (1.1, 1.2) weisen erstaunlicherweise sowohl mit als auch ohne Einsatz von NaHCO₃ im Medium die gleichen Produktkonzentrationen auf. Bei den Läufen mit Einsatz von Sod-ß sind die erreichten Produktkonzentrationen ebenfalls nicht von der eingesetzten Menge an NaHCO₃ im Medium abhängig, da in allen Läufen (1.3-1.6) eine ähnliche maximale Produktkonzentration an Tag 11 erreicht wird (siehe Abbildung 6).

Im CO₂-geregelten Fermentationsprozess ist der Einsatz von NaHCO₃ im Medium überraschenderweise nicht erforderlich. Ohne NaHCO₃ im Medium wird die CO₂-Regelung im Prozess erleichtert, da durch das Medium kein CO₂ gebildet werden kann.

### Resultat 7:

Es konnte überraschenderweise gezeigt werden, dass ein niedriger CO₂ Partialdruck zu Anfang der Fermentation ein kritischer Faktor für ein optimiertes Fermentationsergebnis ist.

Einen Fermentationsprozess mit einem hohen pCO₂ zu starten ist dagegen nicht von Vorteil.

Sowohl die Lebendzellkonzentrationen als auch die Produktkonzentrationen aller Läufe außer des Laufes 3.2 mit unterschiedlichen pCO₂-Profilen verlaufen ähnlich und erreichen ungefähr die gleichen Maxima (siehe Abbildung 7A und 7B). Im Lauf 3.2 wachsen die Zellen schlechter und erreichen deshalb eine niedrigere Produktkonzentration.

Bei Betrachtung der zugehörigen pCO₂-Werte in Abbildung 7C ist ein wesentlicher Unterschied des Laufes 3.2 im Vergleich zu allen anderen Läufen ersichtlich. Der pCO₂ an Tag 1 liegt im Lauf 3.2 über 8%. Dahingegen liegen die pCO₂-Werte der anderen Läufe an Tag 1 alle unter 5%.

Durch einen anfänglich hohen pCO₂ wird das Zellwachstum gehemmt.

### Resultat 8:

Die in Abbildung 8 dargestellten Lebendzellkonzentrationen zeigen, dass das Zellwachstum durch die Zugabe von Natriumacetat (SF5 und 6) beeinträchtigt wird. Die Lebendzellzahlen steigen mit dem Einsatz von Natriumacetat nur leicht an und fallen bereits drei Tage nach Zugabe wieder ab, sodass von einer toxischen Wirkung des Natriumacetats ausgegangen werden kann. Aufgrund der nachgewiesenen Zelltoxizität wird die Essigsäure nicht als bevorzugte Säure für die pH-Regelung im Fermentationsprozess eingesetzt.

Alle anderen Salze zeigen keine toxischen Wirkungen auf das Zellwachstum und können zum Test im Fermentationsprozess eingesetzt werden. Bevorzugt werden Salzsäure, Schwefelsäure und Phosphorsäure.

### Resultat 9:

Durch die Zugabe von Salzsäure (4.3), Schwefelsäure (4.4) und Phosphorsäure (4.5) werden im Fermentationsprozess gleiche Produktkonzentrationen erreicht wie Abbildung 9B zeigt.

Bei Betrachtung der Lebendzellkonzentrationen dieser Ansätze (siehe Abbildung 9A) fällt auf, dass im Lauf 4.3 mit Zugabe von Salzsäure eine niedrigere Zellzahl als in den anderen Läufen erreicht wird. Da die Produktkonzentration jedoch genauso hoch ist wie in allen anderen Läufen, ist die Produktivität der Zellen durch Zugabe der Salzsäure höher.

Im Lauf 4.4 mit Zugabe von Schwefelsäure entsteht ab Tag 7 weniger Laktat als in den anderen Läufen wie Abbildung 9C zeigt. Da Laktat ab einer gewissen Konzentration toxisch auf die Zellen wirkt, ist eine niedrige Laktatkonzentration im Prozess von Vorteil.

Sowohl die Schwefelsäure als auch die Salzsäure sind im Fermentationsprozess von Vorteil. Die Schwefelsäure sorgt für eine geringe Laktatproduktion und die Salzsäure sorgt für eine hohe Produktivität der Zellen.

### Resultat 10:

Beim Vergleich des Standardprozesses (4.1) mit den CO₂-geregelten Prozessen mit Einsatz verschiedener Säuren (4.3 - 4.5) ergeben sich in allen Läufen ähnliche Produktkonzentrationen (siehe Abbildung 10). Im Lauf 4.5 mit Zugabe von Phosphorsäure wird annähernd die gleiche maximale Produktkonzentration erreicht wie im Lauf 4.1 (Standardprozess).

Der CO₂.geregelte Prozess bringt im Vergleich zum Standardprozess keine Nachteile im Bezug auf die Produktkonzentration mit sich. Bei weiteren Optimierungen des CO₂-geregelten Prozesses kann die Produktkonzentration im Vergleich zum Standardprozess sogar gesteigert werden.

Zusammengefasst zeigen die Ergebnisse eine vergleichbare Performance bezüglich Zellwachstum und Produktivität und sogar bessere Performance durch niedrigere Laktatbildung wenn die getesteten Säuren zur pH Regelung eingesetzt werden.

### Resultat 11:

Um das Ergebnis der Prozessperformance für unterschiedliche pCO₂-Profile, wie in Resultat 7 beschrieben, auf eine breitere Datenbasis zu stellen, wird in Beispiel 9 ein weiterer Versuchsansatz mit unterschiedlichen pCO₂-Profilen, diesmal mit einer anderen Modellzelle (CHO) durchgeführt.

Die unterschiedlichen pCO₂-Profile, welche in diesem Versuchsansatz eingestellt werden, werden in Abbildung 11C wiedergegeben.

Wie in den Abbildungen 11A und 11B ersichtlich ist, hebt sich der Fermentationslauf KF1 sowohl für das Zellwachstum als auch für den Verlauf der Produktkonzentration deutlich von der restlichen Kurvenschar ab. Dieser Fermenterlauf (KF1) wurde mit einem pCO₂-Profil von Tag 0 bis Tag 11 bei 3% pCO₂ betrieben.

Mit diesem Versuchsansatz konnte somit bestätigt werden, dass der CO₂ Partialdruck zu Anfang der Fermentation (Anwachsphase, speziell Tag 0 bis 3) ein wichtiger Faktor für ein optimiertes Fermentationsergebnis darstellt.

Beispiel 9 bestätigt, dass ein gezielt geregelter niedriger CO₂ Partialdruck zu Anfang der Fermentation sowohl für das Zellwachstum als auch für den Verlauf der Produktkonzentration das beste Ergebnis liefert. Dies gilt auch bzw. insbesondere bei Verwendung HCO₃⁻ bzw. CO₃²⁻ Ionen reduzierter Kultivierungsmedien (insbesondere unter 12mmol/L bzw. bei 8mmol/L HCO₃⁻ bzw. CO₃²⁻ Ionen) bzw. bei Verwendung komplett HCO₃⁻ bzw. CO₃²⁻ Ionen freier Kultivierungsmedien.

### DEFINITIONEN

Bevor die Erfindung nachfolgend anhand nicht-beschränkender Ausführungs-beispiele näher erläutert wird, sei darauf verwiesen, dass die Verwendung unbestimmter Artikel, beispielsweise "ein" oder "eine", sowie die bestimmter Artikel, wie "der", "die", "das" Einzahl u n d Mehrzahl des entsprechenden Begriffs einschließen, es sei denn, dass eine der beiden Formen explizit ausgeschlossen und auf eine bestimmte Form (Einzahl, Mehrzahl) verwiesen wird. So schließt der Begriff "eine Zelle" automatisch "mehrere Zellen" mit ein, es sei denn es wird explizit darauf verwiesen, dass nur eine einzige Zelle gemeint ist. Einzahl ist beispielsweise dort explizit gemeint, wo "ein" durch (1) ergänzt ist.

Unter einem "biopharmazeutischen Produktionsprozesses" versteht man die Produktion von Biomolekülen mittels eukaryotischen Zellen durch einen Fermentationsprozess. Dies umfasst insbesondere die Produktion von Proteinen wie Antikörpern in Säugerzellen wie CHO, NS0, PERC.6 Zellen.

Unter einem Biomolekül versteht man insbesondere ein Protein von Interesse bzw. ein DNA oder RNA Molekül wie beispielsweise RNAi, anti-sense RNA etc.

Die Optimierung eines biopharmazeutischen Produktionsprozesses meint die Anpassung der Medien und physikalischen Parameter im Fermentationsprozess. Diese umfassen beispielsweise pCO₂, Rührerdrehzahl etc. Mit der Optimierung wird beispielsweise die Steigerung des Titers verbessert oder die Produktqualität (z.B. verändertes Glykosylierungsmuster des Proteins oder andere posttranslationale Modifikationen eines Proteins) oder die Robustheit des Prozesses (Unempfindlichkeit gegenüber kleineren Prozessschwankungen, kleine Änderung hat keine Auswirkung auf den Prozess).

Der Begriff "nicht CO₂-bildende Säure und/oder Lauge" meint Säuren und Laugen, die durch chemische Reaktion kein CO₂ abgeben, z.Bsp. NaOH, HCl, H₃PO₄, CH₃COOH, H₂SO₄.

"Regulierung des pCO₂" bedeutet, dass der CO₂-Partialdruck in der Fermentationslösung auf einem Sollwert gehalten / eingestellt wird. Dies geschieht beispielsweise über Zufuhr von CO₂, O₂, N₂ und/oder Luft während des Bioprozesses. In der vorliegenden Erfindung erfolgt die "Regulierung des pCO₂" bevorzugt durch Begasung mit CO₂ und /oder N₂.

"Komplett HCO₃- bzw. CO₃²⁻ Ionen frei" bedeutet, dass das von Tag 0 an bis zum Ende der Fermentation eingesetzte Medium HCO₃⁻ bzw. CO₃²⁻ Ionen frei ist. Es werden dem Medium von Tag 0 an bis zum Ende der Fermentation auch keine HCO₃⁻ bzw. CO₃²⁻ Ionen über separate Zugaben ("Feeds") zugeführt.

Unter einer "Fed-Batch" Fermentation / einem "Fed-Batch" Verfahren bzw. einem "Fed-Batch" Prozess versteht man einen Fermentationsprozess, welcher durch einen Zustrom an Substraten bis zum maximalen Füllstand betrieben wird. Der Begriff Fed-Batch leitet sich vom englischen fed "gefüttert" und batch "Stapel" ab. Manchmal wird statt dem Begriff Fed-Batch Verfahren auch der Begriff Zulauf-Verfahren verwendet. Der Begriff "Fed-Batch" Fermentation / "Fed-Batch" Verfahren bzw. "Fed-Batch" Prozess ist ein in der Verfahrenstechnik etablierter Begriff. Man bezeichnet damit Prozesse, die als "Stapel", das heißt nacheinander, abgearbeitet werden und durch einen Zustrom (Zufütterung) an Edukten (Ausgangsstoffe) bis zum maximalen Füllstand betrieben werden.

Im Rahmen der vorliegenden Erfindung wird die "Fed-Batch" Fermentation / das "Fed-Batch" Verfahren bzw. der "Fed-Batch" Prozess bevorzugt in drei (3) Abschnitte unterteilt: Anwachsphase, Wachstumsphase, Absterbephase. Die Dauer einer "Fed-Batch" Fermentation ist zeitlich beschränkt. Bevorzugt beträgt die gesamte Dauer der "Fed-Batch" Fermentation / des "Fed-Batch" Verfahrens bzw. des "Fed-Batch" Prozesses 8-15 Tage, besonders bevorzugt 11 Tage.

Die "Fed-Batch" Fermentation / das "Fed-Batch" Verfahren bzw. der "Fed-Batch" Prozess unterscheidet sich von anderen Kultivierungsverfahren wie der Perfusionskultur bzw. Fermentation im Perfusionsverfahren oder der kontinuierlichen Kultivierung. Bei der Perfusionskultur wird der Inkubationsreaktor mit der Zellkultur ständig von einem Mediumfluss durchspült. Dies stellt gleich bleibende Konzentrationsverhältnisse der Nährstoffe, Wachstumsfaktoren, Antibiotika etc., den Abtransport von Stoffwechselendprodukten sowie Nachahmung natürlicher physiologischer und metabolischer Umgebungsbedingungen (Blutkreislauf, Diffusion und Kreislauf von Gewebeflüssigkeit) sicher.

Die "spezifische Produktivität" oder "spezifische Produktbildungsrate" einer Zelle gibt die Menge eines bestimmten Proteins an, die von einer Zelle pro Zeiteinheit produziert, d.h. bei sekretierten Proteinen ins Medium abgegeben wird. Die spezifische Produktivität berechnet sich aus dem Quotienten der Konzentration des Produktes im Medium (=Titer, bestimmt, z.B. über ELISA) und der Anzahl der über den betrachteten Zeitraum vorhandenen produzierenden Zellen, auch bezeichnet als "IVC" (Integral der Lebendzellzahl über die Zeit). Die spezifische Produktivität wird üblicherweise in ,pcd' (= pg/cell*day = Picogramm sezerniertes Protein pro Zelle und Tag) angegeben). "IVC" (Integral der Lebendzellzahl über die Zeit). Die spezifische Produktivität wird üblicherweise in ,pcd' (= pg/cell*day = Picogramm sezerniertes Protein pro Zelle und Tag) angegeben).

Der Begriff "serumfrei" meint Kulturmedien wie auch Kultivierungsbedingungen, die dadurch gekennzeichnet sind, dass Zellen in Abwesenheit von tierischem und/oder humanem Serum, alternativ in Abwesenheit von jeglichen aus Serum isolierten Proteinen, alternativ in Abwesenheit von nicht rekombinant gewonnenen Proteinen kultiviert werden. Folglich meint der Ausdruck "an serumfreie Bedingungen adaptierte Zellen" solche Zellen, die in Abwesenheit von tierischem oder humanem Serum bzw. Serumproteinen vermehrt werden können.

Der Begriff "proteinfrei" meint, dass das Kulturmedium keine tierischen Proteine enthält, wobei aus Bakterien, Hefen oder Pilzen isolierte Proteine nicht als tierische Proteine verstanden werden.

Der Begriff "chemisch definiert" beschreibt ein Zellkulturmedium, welches serumfrei, optional auch proteinfrei ist, und das aus chemisch definierten Substanzen besteht. Chemisch definierte Medien bestehen somit aus einer Mischung aus vorwiegend reinen Einzelsubstanzen. Ein Beispiel für ein chemisch definiertes Medien ist beispielsweise das CD-CHO-Medium der Firma Invitrogen (Carlsbad, CA, US).

Der Ausdruck "eine in Suspension kultivierbare Zelle" bezieht sich auf Zellen, die an das Wachstum in flüssigen Kulturen ("Suspensionskulturen") adaptiert sind und deren Fähigkeit zu Adhäsion an Gefäßoberflächen, beispielsweise an Zellkulturschalen / -flaschen eingeschränkt bzw. verloren gegangen ist. Zellen, die sowohl an serumfreies Wachstum als auch an das Wachstum in Suspension adaptiert sind, werden als "an serumfreies Medium adaptierte und nicht adhärente Zellen" bezeichnet.

Der Begriff "Gen von Interesse" oder "rekombinantes Gen von Interesse" umfasst eine Nukleotidsequenz beliebiger Länge, die für ein Produkt von Interesse kodiert. Das Genprodukt oder auch "Produkt von Interesse" ist in der Regel ein Protein, Polypeptid, Peptid bzw. Fragment oder Derivat davon. Es kann aber auch RNA oder antisense RNA sein. Das Gen von Interesse kann in voller Länge, in verkürzter Form, als Fusionsgen oder markiertes Gen vorliegen. Es kann sich um genomische DNA oder vorzugsweise cDNA bzw. entsprechende Fragmente oder Fusionen handeln. Das Gen von Interesse kann die native Gensequenz darstellen, mutiert oder auf sonstige Weise modifiziert sein. Derartige Modifikationen schließen Codon-Optimierungen zur Anpassung an eine bestimmte Wirtszelle und eine Humanisierung ein. Das Gen von Interesse kann z.B. für ein sekretiertes, zytoplasmatisches, kernlokalisiertes, membrangebundenes oder zelloberflächengebundenes Polypeptid kodieren.

Der Begriff "Protein von Interesse" bezieht sich auf biopharmazeutisch bedeutsame Proteine/Polypeptide umfassend z.B. Antikörper, Enzyme, Cytokine, Lymphokine, Adhäsionsmoleküle, Rezeptoren sowie deren Derivate bzw. Fragmente. Ein Protein / Produkt von Interesse ist aber nicht auf diese Beispiele beschränkt. Im Allgemeinen sind alle Polypeptide bedeutsam bzw. von Interesse, die als Agonisten oder Antagonisten wirken und/oder therapeutische oder diagnostische Anwendung finden können. Andere Proteine von Interesse sind beispielsweise Proteine/Polypeptide, die zur Veränderung der Eigenschaften von Wirtszellen im Rahmen des sogenannten "Cell Engineering" verwendet werden, wie z.B. anti-apoptotische Proteine, Chaperone, Stoffwechselenzyme, Glykosylierungsenzyme sowie deren Derivate bzw. Fragmente, sind aber nicht auf diese beschränkt.

Der Ausdruck "Polypeptide" wird für Aminosäuresequenzen oder Proteine verwendet und bezeichnet Polymere von Aminosäuren beliebiger Länge. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann z.B. durch Substitutionen, Deletionen oder Insertion von Aminosäuren, Fusion mit anderen Proteinen, unter Beibehaltung seiner biologischen Aktivität modifiziert werden. Zudem können die Polypeptide multimerisieren und Homo- und Heteromere bilden.

Unter "rekombinanten Proteinen" versteht man Proteine, welche durch rekombinante Expression in Wirtszellen produziert werden. Solche rekombinanten Proteine werden unter höchsten Reinheitsauflagen produziert, um das Kontaminationsrisiko möglichst gering zu halten. Rekombinante Proteine werden üblicherweise in passenden Wirtszellen produziert wie z.B. Hefezellen, tierische Zellen oder prokaryotische Zellen (E. coli oder andere Bakterienstämme) indem ein Expressionsvektor wie beispielsweise ein Plasmid, Bakteriophage, nackte DNA oder ein Virus benutzt wird, um das rekombinante Protein in die Wirtszelle einzuführen.

Ein rekombinantes Protein wird von einem rekombinanten Gen kodiert und von einer rekombinanten Zelle exprimiert.

Rekombinante Proteine werden üblicherweise gereinigt als konzentrierte Protein-Lösungen oder in Pulverform kommerziell angeboten. Rekombinantes HSA ist beispielsweise von diversen kommerziellen Anbietern wie Sigma Aldrich erhältlich. Beispiele für therapeutische Proteine sind Insulin, Insulin-ähnlicher Wachstumsfaktor, humanes Wachstumshormon (hGH) und andere Wachstumsfaktoren, Rezeptoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (single chain) Antikörper und Fragmente davon, wie z.B. Fab, Fab', F(ab')2, Fc und Fc'-Fragmente, leichte (L) und schwere (H) Immunglobulinketten und deren konstante, variable oder hypervariable Regionen sowie Fv- und Fd-Fragmente. Die Antikörper können humanen oder nichthumanen Ursprungs sein. Auch humanisierte und chimäre Antikörper kommen in Frage. Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')2-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10-30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als single-chain Fv-Fragment (scFv) bezeichnet. Beispiele von scFv-Antikörpern sind bekannt und beschrieben.

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen ("coiled coil structure") wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur. Homodimeren durch Überlagerung von VH/VL-Ketten. Die Diabodies können zusätzlich durch eingeführte Disulfidbrücken stabilisiert werden. Beispiele von Diabodies finden sich in der Literatur. Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region.

Mit "Triabody" bezeichnet der Fachmann ein trivalentes homotrimeres scFv-Derivat. Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt.

Der Begriff "Antikörper-Fusion" oder "Antikörper-Fusionsprotein" bezeichnet die Fusion/Kopplung eines Proteins mit einem Antikörper oder einem Teil eines Antikörpers. Insbesondere zählen dazu gentechnisch hergestellt Fusionsproteine, bei denen ein therapeutisches Protein an den Fc-Teil eines Antikörpers gekoppelt wird, um auf diese Weise eine die Halbwertszeit / Stabilität des Proteins im Serum zu erhöhen. Der Begriff umfasst auch Antikörper-Fusionen, die aus einem Peptid und einem Antikörper oder einem Teil eines Antikörpers bestehen.

Im Rahmen der Erfindung bevorzugte Wirtszellen sind Hamsterzellen wie z.B. BHK21, BHK TK-, CHO, CHO-K1, CHO-DUKX, CHO-DUKX B1 und CHO-DG44 Zellen oder Derivate/Abkömmlinge dieser Zelllinien. Weitere Wirtszellen sind CHO-DG44, CHO-DUKX, CHO-K1 und BHK21 Zellen, z.B. auch CHO-DG44 und CHO-DUKX Zellen. Ebenfalls geeignet sind Myelomzellen der Maus, wie z.B. NS0 und Sp2/0 Zellen sowie Derivate/Abkömmlinge dieser Zelllinien.

Beispiele für Hamster- und Mäusezellen, die erfindungsgemäß angewandt werden können, sind in der folgenden Tabelle 1 angegeben. Aber auch Derivate und Abkömmlinge dieser Zellen, andere Säugerzellen, einschließlich aber nicht beschränkt auf Zelllinien von Mensch, Maus, Ratte, Affen, Nagetieren, oder eukaryontische Zellen, einschließlich aber nicht beschränkt auf Hefe-, Insekten- , Vogel- und Pflanzenzellen, können ebenfalls als Wirtszellen zur Produktion von biopharmazeutischen Proteinen verwendet werden.

**Tabelle 1: Bekannte Produktionszelllinien**

| Zelllinie | Hinterlegungsnummer |
|---|---|
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCC CCL-15 |
| 2254-62.2 (BHK-21-Derivat) | ATCC CRL-8544 |
| CHO | ECACC No. 8505302 |
| CHO-K1 | ATCC CCL-61 |
| CHO-DUKX (= CHO duk⁻, CHO/dhfr⁻) | ATCC CRL-9096 |
| CHO-DUKX B1 | ATCC CRL-9010 |
| CHO-DG44 | Urlaub et al., Cell 33[2], 405-412, 1983 |
| CHO Pro-5 | ATCC CRL-1781 |
| Lec13 | (Stanley P. et al, 1984). |
| V79 | ATCC CCC-93 |
| B14AF28-G3 | ATCC CCL-14 |
| HEK 293 | ATCC CRL-1573 |
| COS-7 | ATCC CRL-1651 |
| U266 | ATCC TIB-196 |
| HuNS1 | ATCC CRL-8644 |
| Per.C6 | (Fallaux, F.J. et al, 1998) |
| CHL | ECACC No. 87111906 |

Es können rekombinante Säugerzellen, z.B. Nagerzellen, murine Zellen wie NS0 und Hamsterzellen wie z.B. CHO- oder BHK-Zellen eingesetzt werden.

Wirtszellen sind besonders geeignet, wenn sie etabliert, adaptiert und komplett unter serumfreien Bedingungen kultiviert werden. Weiterhin geeignet sind Wirtszellen, die zusätzlich in Medium etabliert, adaptiert und komplett kultiviert werden, welches nicht nur serumfrei ist, sondern auch noch frei von jeglichen Proteinen /Peptiden tierischen Ursprungs.

Kommerziell erhältliche Medien wie Ham's F12 (Sigma, Deisenhofen, Deutschland), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA), CHO-S-Invtirogen), serumfreies CHO Medium (Sigma), und proteinfreies CHO Medium (Sigma) sind Beispiele passender Nährlösungen.

Der Begriff "Produktionszelle" oder "Produzenten-Zelle" oder "Produktionsklon" bezeichnet eine Zelle, die in einem Prozess zur Herstellung eines Proteins verwendet wird. Insbesondere gehören dazu genetisch-veränderte Zellen, die zur industriellen Produktion von recombinanten Proteinen eingesetzt werden. Der Begriff bezeichnet vor allem genetisch-veränderte eukaryontische Wirtszellen, die ein recombinantes Protein exprimieren und zur Herstellung dieses Proteins eingesetzt werden. Darunter fallen insbesondere monoklonale Zelllinien zur Produktion von therapeutischen Proteinen.

### ERFINDUNGSGEMÄSSE AUSFÜHRUNGSFORMEN

Die Erfindung beschreibt eine Methode zur Optimierung eines biopharmazeutischen Produktionsprozesses umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO3⁻ bzw. CO3²⁻ Ionen enthält, wobei besagtes Zellkulturmedium die Pufferkomponente Sodium-β-glycerophosphatpentahydrat (C₃H₇Na₂O₆P x 5 H₂O, Sod-ß enthält,
c) Regulierung des pH-Wertes über eine nicht CO2-bildende Säure und/oder Lauge, wobei während des Bioprozesses eine Regulierung des pCO₂ mit CO2, O2, N2 und/oder Luftzufuhr oder mittels der Drehzahl des Rührers durchgeführt wird.

Die Erfindung beschreibt eine Methode zur Regulierung des pCO2 umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO3⁻ bzw. CO3²⁻ Ionen enthält, wobei besagtes Zellkulturmedium die Pufferkomponente Sodium-β-glycerophosphatpentahydrat (C₃H₇Na₂O₆P x 5 H₂O Sod-ß) enthält,
c) Regulierung des pH-Wertes über eine nicht CO2-bildende Säure und/oder Lauge, wobei während des Bioprozesses eine Regulierung des pCO₂ mit CO2, 02, N2 und/oder Luftzufuhr oder mittels der Drehzahl des Rührers durchgeführt wird.

Die Erfindung beschreibt weiterhin eine Methode zur Verbesserung der Reproduzierbarkeit von Bioprozessen mit eukaryotischen Zellen dadurch charakterisiert, dass mindestens folgende Parameter kontrolliert werden: pCO2 Profil, 02 Profil, pH-Profil, Temperaturprofil, Drehzahl und
a) wobei die Kultivierung der Zelle im Fed-Batch Fermentationsverfahren in einem Zellkulturmedium erfolgt, welches keine HCO3⁻ bzw. CO3²⁻ Ionen enthält, und
b) wobei besagtes Zellkulturmedium die Pufferkomponenten Sodium-β-glycerophosphat-pentahydrat (C₃H₇Na₂O₆P x 5 H₂O, Sod-ß) enthält,
c) wobei die Regulierung des pH-Wertes über eine nicht CO2-bildende Säure und/oder Lauge erfolgt und
d) wobei während des Bioprozesses eine Regulierung des pCO₂ mit CO2, 02, N2 und/oder Luftzufuhr oder mittels der Drehzahl des Rührers durchgeführt wird.

Die Erfindung beschreibt weiterhin eine Methode zur Herstellung eines rekombinanten Produkts von Interesse umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponenten Sodium-β-glycerophosphatpentahydrat (C₃H₇Na₂O₆P x 5 H₂O, Sod-ß) enthält,
c) Regulierung des pH-Wertes über eine nicht CO2-bildende Säure und/oder Lauge, wobei während des Bioprozesses eine Regulierung des pCO₂ mit CO2, 02, N2 und/oder Luftzufuhr oder mittels der Drehzahl des Rührers durchgeführt wird.

In einer bevorzugten Ausführungsform des erfindungsgemäßen Herstellverfahrens ist das Produkt von Interesse ein Protein, bevorzugt ein Antikörper oder Antikörperfragment oder Fc-Fusionsprotein.

In einer bevorzugten Ausführungsform einer der erfindungsgemäßen Methoden wie soeben beschrieben erfolgt eine Regulierung des pCO₂ ausschließlich durch Begasung mit CO₂ und/oder N₂.

Vom Tag 0 an bis zum Ende der Fed-Batch Fermentation/ des Fed-Batch Verfahrens werden keinerlei HCO₃⁻ bzw. CO₃²⁻ Ionen im Medium zugegeben und/oder eingesetzt.

In einer spezifischen Ausführungsform einer der erfindungsgemäßen Methoden wie soeben beschrieben beträgt die Sod-ß Konzentration zwischen 1mmol/L bis 100mmol/L, 5-50, 15-30 bzw. kleiner gleich 100mmol/L bzw. 25mmol/L beträgt. Eine höhere Sod-ß Konzentration als 100mmol/L ist wegen der steigenden Osmolalität nicht erfindungsgemäß.

In einer spezifischen Ausführungsform einer der erfindungsgemäßen Methoden wie soeben beschrieben ist die Säure und/ oder Lauge gemäß Schritt c) NaOH, HCl, H₃PO₄, oder H₂SO₄ ist, bevorzugt NaOH oder HCl.

In einer spezifischen Ausführungsform einer der erfindungsgemäßen Methoden wie soeben beschrieben ist die eukaryotische Zelle eine Säugerzelle, bevorzugt eine Nagerzelle, bevorzugt eine CHO, PER.C6 oder NS0 Zelle ist.

In einer bevorzugten Ausführungsform einer der erfindungsgemäßen Methoden wie oben beschrieben wird der pCO₂ in der Anwachsphase (Tag 0 bis Tag 3 bzw. Tag 1 bis 3, insbesondere an Tag 1 der Fermentation) auf einen Wert kleiner oder gleich 10% bzw. kleiner oder gleich 8% reguliert, bevorzugt wird der pCO₂ eingestellt auf einen Wert zwischen 2% und 8% bzw. zwischen 3% und 8% bzw. zwischen 5% und 8% bzw. zwischen 3% und 5%, besonders bevorzugt ist ein pCO₂ Wert von 5% bzw. 3%.

In einer weiteren bevorzugten Ausführungsform wird der pCO₂ von Tag 0 bis Tag 11 (bzw. bis Fermentationsende) auf 3% reguliert. In einer weiteren bevorzugten Ausführungsform umfasst die Methode weiterhin, dass der pCO2 Wert zusätzlich wie folgt reguliert wird:
i) ein mittlerer pCO₂ (kleiner oder gleich 12%) in der Wachstumsphase (beispielsweise Tag 3-7 oder Tag 4-8), bevorzugt 5-12% bzw. 8-12%, besonders bevorzugt 8-10% bzw. 5-11% bzw. 3-11%,
ii) ein leicht erhöhter bzw. hoher pCO₂ (größer oder gleich 5%, 8% bzw. 15%) in der Absterbephase (Tag 7 bis 11 oder Tag 9-11 bzw. bis zum Eende der (Fed-Batch) Fermentation / bis zum Ende der Kultivierung der Zelle / ), bevorzugt 15-18% bzw. 5-10% bzw. 15%.

Die Erfindung beschreibt weiterhin ein Zellkulturmedium, welches HCO₃⁻ bzw. CO3²⁻ Ionen enthält, wobei besagtes Zellkulturmedium die Pufferkomponente Sodium-β-glycerophosphat-pentahydrat (C₃H₇Na₂O₆P x 5 H₂O, Sod-ß) enthält.

In einer spezifischen Ausführungsform des erfindungsgemäßen Zellkulturmediums sind die kultivierten Zellen eukaryontische Zellen, insbesondere Säugerzellen, insbesondere Nagerzellen, insbesondere Hamsterzellen, insbesondere CHO Zellen.

In einer spezifischen Ausführungsform des erfindungsgemäßen Zellkulturmediums beträgt die Sod-ß Konzentration zwischen 1mmol/L bis 100mmol/L, 5-50, 15-30 bzw. kleiner gleich 100mmol/L bzw. 25mmol/L beträgt. Eine höhere Sod-ß Konzentration als 100mmol/L ist wegen der steigenden Osmolalität nicht erfindungsgemäß.

Die Erfindung beschreibt weiterhin eine Fed-Batch Fermentations Ausrüstung bzw. einen Satz an Fed-Batch Fermentations Ausrüstungsteilen, auf englisch "kit", umfassend folgende Komponenten:
(a) Zellkulturmedium, welches komplett HCO₃⁻ bzw. CO₃²⁻ Ionen frei ist, wobei besagtes Zellkulturmedium die folgende Pufferkomponente enthält:
(b) Fed-Batch Fermenter,
(c) eukaryontische Zelle, bevorzugt eine Säugerzelle, Nagerzelle, Hamsterzelle, insbesondere bevozugt eine CHO Zelle.

Bevorzugt ist die Zelle in (c) eine rekombinante Zelle. Eine rekombinante Zelle enthält eine rekombinante (beispielsweise eine zellfremde) DNA oder RNA Sequenz, welche bevorzugt exprimiert wird und besonders bevorzugt aufgereinigt und isoliert wird. Bevorzugt exprimiert diese rekombinante Zelle ein Gen von Interesse, insbesondere ein therapeutisches Protein, bevorzugt einen Antikörper, ein Antikörperfragment oder ein Fc-Fusionsprotein.

Alle erfindungsgemäßen Methoden werden synonym als Verfahren bezeichnet. So betrifft die Erfindung beispielsweise ebenso ein Verfahren zur Regulierung des pCO₂ umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponente Sodium-β-glycerophosphatpentahydrat (C₃H₇Na₂O₆P x 5 H₂O, Sod-ß) enthält,
c) Regulierung des pH-Wertes über eine nicht CO₂-bildende Säure und/oder Lauge, wobei während des Bioprozesses eine Regulierung des pCO₂ mit CO₂, O₂, N₂ und/oder Luftzufuhr oder mittels der Drehzahl des Rührers durchgeführt wird.

In einer bevorzugten Ausführungsform einer der erfindungsgemäßen Verfahren wie soeben beschrieben erfolgt eine Regulierung des pCO₂ ausschließlich durch Begasung mit CO₂ und/ oder N₂.

### EXPERIMENTELLER TEIL

### MATERIAL UND METHODEN

### Fermentation

Die durchgeführten Experimente wurden entweder im Bioreaktor mit einem Fermentations-Volumen von 2 L (Einsaatvolumen 1,8 L), in 250 ml Shakeflasks (Einsaatvolumen 50 ml) oder mittels SensorDish Reader® der Firma Presens (Einsaatvolumen 1,5 ml) durchgeführt.

Versuche mit dem Bioreaktor bieten viele Vorteile wie z.B. Datenerfassung in Echtzeit und Archivierung der Prozessparameter, eine etablierte und valide Regelung der Prozessgrößen Temperatur, pH, Begasung (unterschiedliche Anteile von Gasen) und der Rührerdrehzahl. Die erhobenen Daten sind durch die kontrollierten Bedingungen reproduzierbar und können auf größere Bioreaktoren übertragen werden (Scale-up/ Skalierung). Die Ausbeuten im Bioreaktor sind im Vergleich meist höher, als bei alternativen Fermentationsarten. Dies resultiert u. a. aus dem integrierten Rührwerk mit seiner turbulenten Durchmischung welches eine bessere Nährstoffversorgung und einen erhöhten Sauerstoffeintrag ermöglicht. Allerdings ist der Betrieb relativ aufwendig und zeitintensiv, da Anfangs der Aufbau und die Sterilisation der Fermentationsgerätschaften/des Equipments, während der Fermentation tägliche Betreuungsarbeiten und nach der Fermentation der Abbau und eine aufwändige Reinigung erforderlich sind. Der Betrieb ist damit sehr personal- und kostenintensiv.

Bioreaktoren werden dann zur Fermentation eingesetzt, wenn in Vorversuchen bereits gute Ergebnisse erzielt wurden und die zusätzlichen Vorteile eines Bioreaktors gefordert sind.

Bei den im Experimentteil beschriebenen Fermentationen am Bioreaktor wurden Fed-Batch-Prozesse gefahren.

Die Kultivierung von Zellen in Schüttelkolben (Shakeflask) bietet den Vorteil, dass sie mit relativ einfachen Mitteln zu bewerkstelligen ist. Man benötigt neben einem Brutschrank, einer LAF-Box (laminar flow, steriler Arbeitsbereich) und den Einweg-Kolben keine weiteren technischen Einrichtungen. Die Kolben werden unter sterilen Verhältnissen mit Zellkulturmedium und Inoculum einer definierten Konzentration befüllt und anschließend in den Brutschrank überführt. Über die Einstellungsmöglichkeiten am Brutschrank (Solltemperatur, -luftfeuchte, -Drehzahl und -CO₂-Gehalt) kann Einfluss auf die Zellkultur genommen werden. Da die Schüttelkoben steril verpackt vorliegen, ist keine weitere Vorbereitung des Equipments nötig. Die Nachteile liegen jedoch auf der Hand: Es ist keine Online-Datenerfassung, keine Regelung von pH, Begasung und Feed möglich. Der Probenzug für Analytik, Feed- und Boluszugaben muss jeweils unter der LAF-Box erfolgen. Dies ist eine Quelle für Kontaminationen oder Fehler und erfordert neben einem routiniertem Handling sehr viel Zeit. Ferner unterliegt man von Seiten der Analytik her den Einschränkungen des Probenvolumens: Es werden z.B. beim Fermentationsstart 50 ml eingesät, bei jedem (meist täglichen) Probenzug zwei bis drei Milliliter entfernt. Obwohl dies teilweise durch Feed- und Bolusgaben relativiert wird, ergibt sich jedes Mal eine, im Verhältnis zum Gesamtvolumen der Kultur, große Entnahme. Generell eignen sich Versuche in kostengünstigen Shakeflasks auf Grund ihres einfachen Handlings und ihrer guten Auswertungsmöglichkeiten gut als Vorstufe für Bioreaktorversuche.

Experimente mittels SensorDish-Reader (=SDR) vereinen teilweise die Vorteile von Bioreaktoren und Schüttelkolben. Bei diesem Verfahren wird in eine 24-Well Platte mit je nur 1,5 ml Zellsuspension pro Well eingesät und diese mit Deckel verschlossen in den Brutschrank gestellt. Auf der Unterseite jedes Wells ist ein Sensorpunkt aufgetragen. Unter der Well-Platte befindet sich der sog. Reader der diesen Sensor ausliest. Dies erlaubt die nicht invasive Messung von pH oder pO₂ online und in Echtzeit. Es ist keine vorhergehende Kalibration nötig, die Kalibrationsdaten werden zu Beginn anhand der aufgedruckten Batch-Nummer automatisch in die Steuerungssoftware geladen.

Die Möglichkeiten von SDR sind jedoch darauf beschränkt, den pH-Wert im Well zu messen und mittels Interpretationen des pO₂-Abfalls über die Zeit hinweg, Rückschluss auf das Zellwachstum zu nehmen. Das Verfahren bietet sich an, wenn man Toxizitäten, Effekte von Einzelsubstanzen oder Grenzkonzentrationen testen will. Auch bei Versuchen, die eine Mehrfachbestimmung erfordern, ist SDR sinnvoll. Da die Probennahme entfällt und das Experiment nur eine Laufzeit von drei Tagen hat und somit auch die Feedings entfallen, ist diese Methode sehr zeitsparend und vom Handling sehr angenehm.

### pH-Regulation

Bei den Versuchen mit dem SensorDish-Reader und bei der Fermentation in Shakeflasks im Brutschrank wird keine aktive pH-Regulation vorgenommen.

Die pH-Regulation im Bioreaktor erfolgt bei BI regulär über die Zugabe von Na₂CO₃ (aq) und CO₂ (gas). Die Regelung erfolgt automatisch über SimaticIT® (s. Punkt Software). Bei pHᵢₛₜ > pHₛₒₗₗ wird CO₂ (gas) eingegast, bei pHᵢₛₜ < pHₛₒₗₗ wird über eine geregelte Pumpe Na₂CO_{3 (aq)} zudosiert bis der Sollwert, +/- definiertes Totband, wieder erreicht wird.

Im aktuellen Fall wird bei einigen Fermentationen aus beschriebenen Gründen Na₂CO_{3 (aq)} gegen äquivalent wirksame Natronlauge ersetzt.

### Medien

Als Basis Zellkultur-Medium wird bei allen Fermentationen ein BI proprietäres Medium als Standardmedium eingesetzt. Die Medien A und B unterscheiden sich nur durch den voreingestellten pH-Wert. Es handelt sich bei beiden Medien um serumfreie Produktionsmedien, die in der eigenen Medienherstellung just in time produziert werden.

Als Feed-Medium wird ein ebenfalls "in house" hergestelltes BI proprietäres Feed-Medium verwendet. Ein Feed-Medium enthält meist hochreines Wasser (WFI= water for injection), Glucose, Aminosäuren, Wachstumsfaktoren und biologische Extrakte aus z.B. Hefen, die für die jeweilige Zelllinie optimal sind. Das Feed-Medium wird bei den Multifermentationen mittels Pumpe kontinuierlich zugegeben, bei der Fermentationen im Shakeflask per Hand unter der LAF-Box zupipettiert.

Für die Experimente im Bioreaktor wird ein so genannter Multifermenter eingesetzt. Er besteht aus sechs baugleichen Einzelfermentern mit je zwei Litern Fermentationsvolumen, die physisch zu einer fahrbaren Einheit zusammengefügt sind. Die Ansteuerung erfolgt mit PC's über die Software SimaticIT®. Das Grundgerüst des Fermenters bildet der 2L Glasbehälter mit Doppelmantel zur Temperierung und die Deckplatte in die die Sonden und weitere Peripherie wie z.B. Steigrohre eingeschraubt werden.

Bei der Fermentation im Bioreaktor kommen folgende Sonden, die in die Deckplatte des Bioreaktors eingeschraubt sind, zum Einsatz: Temperatursonde, pO₂-Sonden, pH-Sonde, CO₂-Sonde.

Für alle durchgeführten Fermentationen wird eine BI-HEX^{®} -Zelle eingesetzt. BI-HEX^{®} steht für Boehringer Ingelheim High Expression System. Die BI-HEX^{®} -Zelle ist eine CHO Zelle, deren genetisches Konstrukt verändert/ optimiert ist, so dass ein Produkt von Interesse effektiv produziert wird. Bei Bedarf an Zellen wird eine Ampulle mit den entsprechenden CHO- Zellen vom Bereich Inoculum aufgetaut, expandiert und eine Stammhaltung angelegt, um daraus eine Expansion für eine Einsaat in den Bioreaktor oder Shakeflask zu gewährleisten. Die Zelle sezerniert einn bestimmtes Biomolekül, bevorzugt ein Protein, bevorzugt einen bestimmten Antikörper extrazellulär ins Zellkulturmedium. Dieser wird bei der Konzentrationsbestimmung des Titers nachgewiesen.

### BEISPIEL 1

### SDR-Versuch (Reduzierung NaHCO₃-Konzentration)

Die elementare Funktion von NaHCO₃ für die Zelle wurde bereits beschrieben. In diesem Versuch soll gezeigt werden, wie, bzw. ob sich verringerte NaHCO₃-Konzentrationen auf das Zellwachstum auswirken. Die Beurteilung erfolgt mittels Rückschluss über den Sauerstoffverbrauch (durch Zellwachstum), im Zellkulturmedium. Das Experiment wird an einem SensorDish-Reader durchgeführt.

Die Versuchsreihe wurde mit n = 4 durchgeführt, um Ausreißer zu erkennen und die allgemeine Streuung beurteilen zu können. Eingesetzt wird eine 24-well Platte OxiDish OD24. Zusätzlich zu den fünf unterschiedlichen NaHCO₃-Konzentrationen wird eine Versuchsreihe mit einer bestimmten Konzentration NaHCO₃ und HEPES gefahren. Diese Kombination wird in der Literatur beschrieben, dort allerdings nur mit 3 % pCO₂ betrieben. Der aktuelle Versuch (6) wird, um eine Vergleichbarkeit zu anderen Versuchen herzustellen, mit 5 % pCO₂ betrieben. Weiteres siehe Experimentplan Tabelle 1. Um die Varianz so gering wie möglich zu halten, wird die Zellsuspension für eine Versuchsreihe gleicher Konzentration jeweils im Pool hergestellt, in die Wells pipettiert und anschließend jeweilig mit dem berechneten, identischen Volumen an NaHCO₃-Stocklösung versehen.

| | | | | | |
|---|---|---|---|---|---|
| **Plattform:** | SensorDish-Reader (SDR) | **Zelle:** | CHO | **Laufzeit:** | 90 h |
| **Medium:** | MEDIUM A w/o NaHCO₃ + Einwaage/Stocklösung Puffersubstanz | | | | |
| **Feeding:** | -entfällt- | **T/L:** | 36,8°C / 70% | | |
| **CO₂-Profil:** | konstant 5 % | **Einsaatdichte:** | 0,6 | | |

| **Versuchsreihe Nummer** | **Puffersubstanz (en) / Konzentration** | **Wiederholungen (n)** |
|---|---|---|
| 1 | NaHCO₃ / A | 4 |
| 2 | NaHCO₃ / B | 4 |
| 3 | NaHCO₃ / C | 4 |
| 4 | NaHCO₃ / D | 4 |
| 5 | NaHCO₃ / D + HEPES / E | 4 |
| 6 | Kontrolle MEDIUM A (NaHCO₃-haltig) / F | 4 |

**Tabelle 1**

| NaHCO₃ | | | HEPES | |
|---|---|---|---|---|
| mmol/L | Normierte Angabe | | mmol/L | Normierte Angabe |
| 0 | A | | 0 | A |
| 1 | B | | 20 | E |
| 4 | C | | | |
| 8 | D | | | |
| 36 mmol | (Ktr) / F | | | |

Bei Versuchsreihe Nummer fünf wurde neben NaHCO₃ auch HEPES mittels Stocklösung zum Pool beigegeben.

Zur Auswertung wird das Diagramm in Abbildung 1 herangezogen, bei dem die pO₂-Werte jedes Wells gegen die Zeit aufgetragen sind. Das Diagramm ist bereits von einem Ausreißer in Versuchsreihe vier bereinigt und basiert auf den arithmetischen Mittelwerten der 4-fachen (bei Versuchsreihe vier, 3-fachen) Wiederholung.
-> Die Reduzierte NaHCO₃ Konzentration von 8mmol/L zeigt ein vorteilhaftes, der Kontrolle ähnliches pO₂ Profil (Zellwachstum).

### BEISPIEL 2

### Shakeflaskversuch

Die Auswahl der Puffersysteme (zellfrei) wird nun mit Zellen betrieben. Kriterium für die Auswahl der in diesem Versuch verwendeten Puffersubstanzen ist eine akzeptable Osmolalität und gutes Pufferverhalten bei der Begasung im Schüttelinkubator.

Mit in das Versuchssetup / Experimentplan geht die Erkenntnis aus Beispiel 1 ein, dass auch mit einer auf Konzentration D (=8mmol/L) reduzierten NaHCO₃-Konzentration gute Wachstumsraten (gefolgert aus dem Sauerstoffverbrauch) erzielt werden können.

Ziel ist die Ermittlung des, auf die Wachstumsperformance der Zelle bezogenen, besten Puffermediums als Basis für eine folgende Fermentation im Bioreaktor.

Die Herstellung erfolgt analytisch so exakt wie möglich. Die pH-Werte werden mittels BGA-Abgleich eingestellt. Geringe Pufferkonzentrationen werden durch Stocklösungen zugegeben. Nach der Herstellung erfolgt eine Sterilfiltration. Zur Absicherung werden die Versuche mit n = 2 ausgeführt. Um zwei unterschiedliche Begasungsvarianten zu realisieren, erfolgt eine Aufsplittung auf zwei unterschiedliche Brutschränke. Eine Zusammenfassung ist dem Experimentplan zu entnehmen.

| | | | | | | | |
|---|---|---|---|---|---|---|---|
| **Plattform:** | Shakeflasks im Brutschrank | | | **Zelle:** | CHO d | **Laufzeit:** | 11 |
| **Medium:** | MEDIUM A w/o NaHCO₃ | | | w/o MOPS + Einwaage/Stocklösung Puffersubstanz | | | |
| **Feeding:** | 1,5 ml / Tag | **T/L:** | 37°C / 70% | **CO₂-Profil:** | s. Experimentplan | **Einsaatdichte:** | 1,1 |

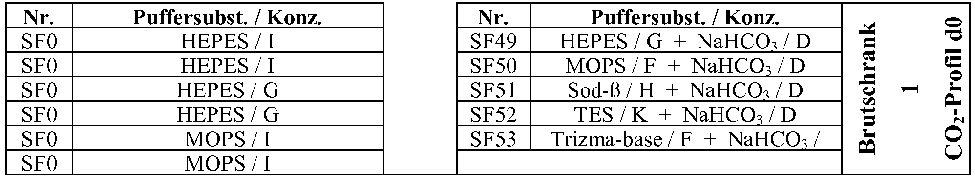

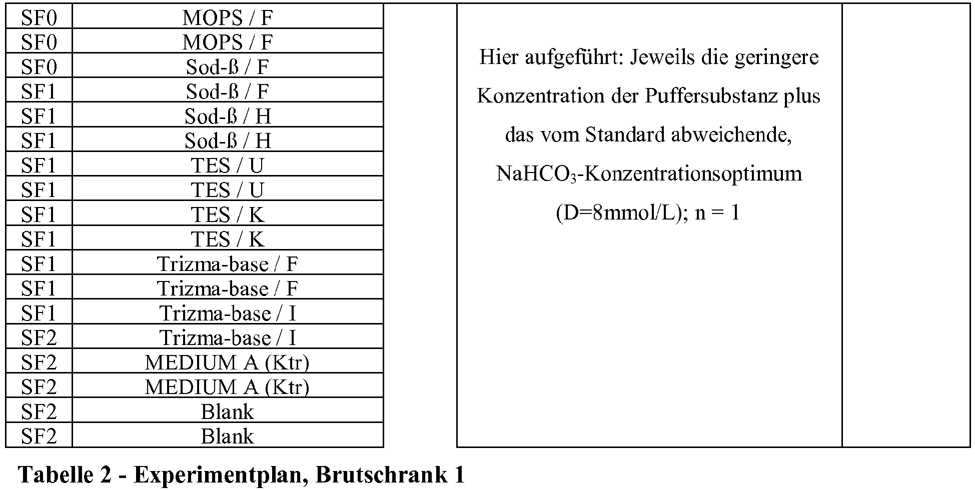

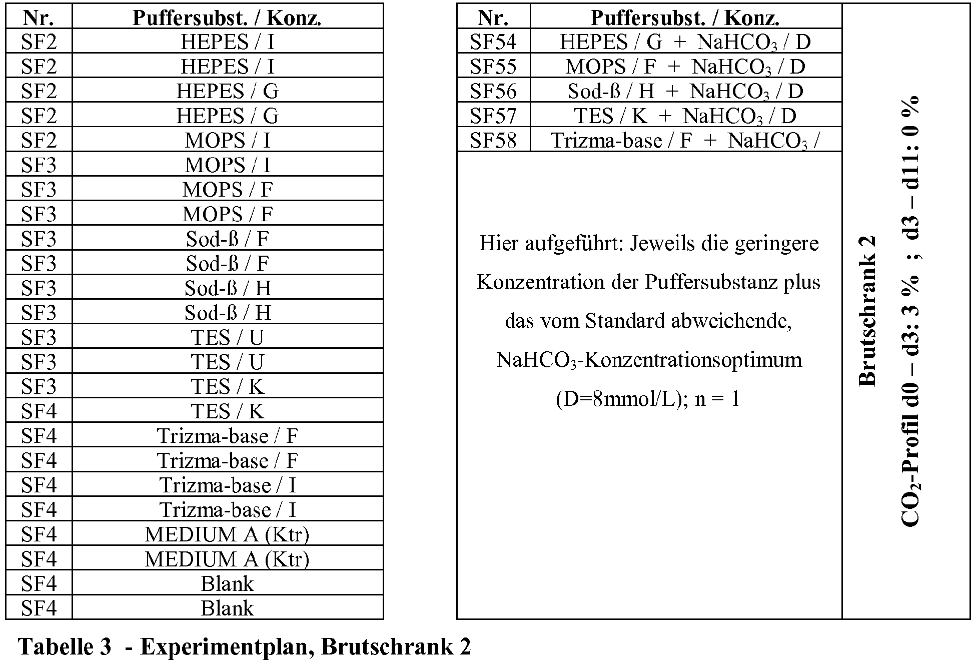

Die Analytik an d0 (Tag 0) erfolgte sofort nach der Einsaat unter der LAF-Box, d.h. ohne vorherige Einwirkung der CO₂-Atmosphäre des Brutschrankes.

In Abbildung 2A ist ersichtlich, dass alle Puffermedienansätze bis auf Trizma-base (SF19 und SF20) mit Konzentration I Zellwachstum zeigen. Das beste Wachstum zeigen die beiden Kontrollen (SF21 und SF22), doch ist der Unterschied zu den Puffermedienansätzen, zumindest bis d8, relativ klein. Die drei besten Puffer, rangierend bei einer Zelldichte um 45, sind Sod-ß / H; TES / K + NaHCO₃ / D und MOPS / F + NaHCO₃ / D. Trizma-base läuft in jeglicher Konzentration schlecht, der pH ist zwar stabil aber evtl. liegt eine Toxizität vor.

In Abbildung 2B ist der Maßstab von Abbildung 2A beibehalten worden. Dabei sieht man deutlich, dass die erreichten Zelldichten bei diesem CO₂-Profil wesentlich geringer ausfallen. Die Kontrollen schneiden zwar auch hier am Besten ab, doch ist eine maximale Zelldichte von 20 nicht akzeptabel. Die zwei besten Pufferansätze, rangierend bei einer Zelldichte um 18, sind hier Sod-ß / H + NaHCO₃ / D und MOPS / F. Aus diesem Grund beschränkt sich die weitere Auswertung primär auf die Shakeflasks aus Brutschrank 1 mit dem dazugehörigen CO₂-Profil.

Generell ist die Kombination mit NaHCO₃ / D bei den niedrigen Pufferkonzentrationen vorteilhaft.

Bei der vorgenommenen pCO₂ Messung wird festgestellt, dass das vorgegebene pCO₂ Profil eingehalten wird. Es ist ersichtlich, dass je später der Zeitpunkt des Probenzuges ist, desto tiefer der gemessene pCO₂. Dies ist ein Resultat des mehrmaligen Öffnens des Brutschrankes.

Die Kontrollen zeigen bei Brutschrank 1 gegen Ende erneut einen Anstieg des pCO₂, welcher vermutlich aus der Eigenproduktion der Zellen resultiert.

Auch der pH-Verlauf über die Dauer der Fermentation hinweg wird gemessen. Dabei sind die pH-Werte auf akzeptablem Niveau. Die Kontrollen (SF21 und SF22) liegen am Tag 2 (d2) noch über dem eigentlichen Startwert von 7,00 +/- 0,05. Dies könnte an der Abweichung vom pH-Meter gegenüber dem BGA liegen; die Kontrolle wurde direkt ohne Eingriff aus der Medienherstellung bezogen, wo der pH-Wert ohne BGA eingestellt wird. Bei den Kontrollen sinkt der pH-Wert am Ende am stärksten ab, basierend auf der Ansäuerung der Zellkultur.

In Abbildung 2C sind die erreichten Titerkonzentrationen (normiert) aufgetragen. Bis auf wenige Ausnahmen ist ein ansteigender Titer zwischen d8 und d11 erkennbar. Der Zugewinn ist dabei bei den Shakeflasks SF49 bis SF52, den Medien mit der reduzierten NaHCO₃-Konzentration, sogar mehr als zehnfach!

Bemerkenswert ist auch die Tatsache, dass erstmals mit einer neuen Puffersubstanz im Medium die herkömmliche Kontrolle (SF21 u. SF22) überholt wurde: SF52 mit TES / K + NaHCO₃ / D liefern am d11 mit norm. Produktkonzentration 83,6 ein sehr gutes Ergebnis.

### Resultat:

→ Identifizierung der besten Puffersubstanzen bei NaHCO₃ freiem Zellkulturmedium (linke Tabellenhälfte) bzw. bei NaHCO₃ erniedrigtem (=8mmol/L) Zellkulturmedium (rechte Tabellenhälfte).
→ Die besten Puffersubstanzen waren: TES und Sod-ß

### BEISPIEL 3 BIOREAKTOR-FERMENTATION

### Versuchsaufbau pCO₂ Regulierung:

Zur Regelung eines Prozesses stehen unterschiedliche Reglertypen in unterschiedlichen Kombinationen zur Verfügung. Sie können folgende Regleranteile aufweisen:
1. P-Regler: Proportional-Regler. Sie finden Einsatz bei Anwendungen mit geringen Anforderungen an die Regelgenauigkeit. P-Regler weißen eine bleibende Regelabweichung auf.
2. I-Regler: Integral-Regler. Sie finden Einsatz bei Anwendungen mit geringen Anforderungen an die Regelgeschwindigkeit, regeln aber sehr genau.

In der Praxis am häufigsten und auch bei der vorliegenden Fermentationssteuerung werden PI-Regler eingesetzt. PI-Regler kombinieren die Vorteile von P- und 1-Reglern: Sie regeln zügig durch den P-Anteil und genau und ohne bleibende Regelabweichung durch den I-Anteil; die Wirkungen der Einzelregler werden addiert.

Das Regelverhalten soll im anschließenden Verlauf der Fermentation durch das Abändern der Regeleinstellungen (P-I Anteile) optimiert werden.

Der Bioreaktor enthält folgende Komponenten:

**Tabelle 4**

| Nr. | Bezeichnung | Funktion |
|---|---|---|
| 1 | Temperatur-Sonde | Messung der Temperatur im Bioreaktor. |
| 2 | Begasungsfilter | Sterilbarriere zwischen einströmenden Gasgemisch und Bioreaktor. |
| 3 | Begasungsrohr | Düse am untere Ende setzt Gasblasen frei. |
| 4 | Abluftstutzen | Entlüftung des Bioreaktors. |
| 5 | Oberflächenzugabeport 1 | Über 3-fach Verteiler werden organische Lösungen wie Glucose, Glutamin und Feed zugegeben. |
| 6 | Zell- & PBS - Zugabeport | Befüllen des Bioreaktors mit Inoculum und Puffer. |
| 7 | Oberflächenzugabeport 2 | Über 3-fach Verteiler werden chemische Lösungen wie Antischaum und pH-Agens zugegeben |
| 8 | Probenport | Probenzug mittels steriler Einmalspritze. |
| 9 | CO₂-Sonde | Messung des pCO₂ im Bioreaktor. |
| 10 | O₂-Sonde | Messung des pO₂ im Bioreaktor. |
| 11 | pH-Sonde | Messung des pH-Wertes im Bioreaktor. |

Der veränderte Bioreaktor enthält insbesondere eine integrierter CO₂ Sonde. Das Hardware-Setup der Experimente ist jedoch prinzipiell variabel, um verschiedenen Anforderungen zu entsprechen. Zum Beispiel kann auf ein Steigrohr verzichtet werden und dafür eine weitere Sonde eingebaut oder weitere/andere Lösungen mittels Schlauchpumpen automatisch zugegeben werden.

Bei den pCO₂ Messungen kann man zunächst erkennen, dass der Regler nach Sollwertänderungen lange Zeit schwingt. Ab dem Zeitpunkt der Einsaat bis zum Erreichen des Sollwertes von 8 % schwingt der Regler rund zwei Tage. Bei der Absenkung des Sollwertes von 8% auf 6% schwingt die Regelung noch 14 Stunden; nach einer In-Prozess-Kalibration ca. 12 Stunden.

Gemessen werden auch die Gas-Volumenströme, die bei der maximalen negativen Stellgröße (-45 %) des pCO₂-Reglers anliegen. Trotz dieser Stellgröße wird der Sollwert von 2 % pCO₂ um + 0,3 % nicht erreicht. Daraus ist abzuleiten, dass mit einem pCO₂-Sollwert von 2% eine kritische Untergrenze erreicht wird, welche im Prozess nicht sinnvoll erscheint. Da der eingetragene Stickstoff den Sauerstoff austreibt, muss dies mit einem erhöhten Sauerstoff-Volumenstrom ausgeglichen werden. Der sich aufsummierende Gesamtgas-Volumenstrom kann bzgl. der Schaumbildung eine kritische Größe erreichen. Deutlich wird auch, dass sich nach dem Einpendeln der Regelung auf ein höheres Niveau eine gleichmäßige CO₂-Begasung einstellt.

In Abbildung 3 werden die pCO₂-Online-Daten (entspricht den minütlich archivierten pCO₂-Sonden Messwert) mit den extern nach Probenzug am BGA gemessenen Werten verglichen. Die erste Kalibration ist wenig erfolgreich, da danach eine konstante Differenz auftritt. Nach der zweiten Kalibration jedoch sind die darauf folgenden Messwerte übereinstimmend. Man kann daraus den Schluss ziehen, dass eine In-Prozess-Kalibration erst dann Sinn macht, wenn sich das oszillierende Regelverhalten gelegt hat. Im zweiten Viertel der Fermentation sind die externen Messwerte höher. Die Hypothese hierzu ist, dass zu diesem Zeitpunkt der Zellstoffwechsel hochaktiv ist und dabei sehr viel CO₂ gebildet wird. In der nicht mehr begasten Probe in der Spritze kann dann CO₂ bis zur Messung kummulieren.

Die Abbildung 3 zeigt somit grafisch den Verlauf der internen pCO₂ Messung (Sonde mit automatischer Datenarchivierung) und des externen Messwertes am Blutgasanalyseautomat. Die mit einem Pfeil markierte erste Kalibration (Abgleich zwischen CO2 Sonde und BGA) an Tag 0 (d0) zeigt Der Fermentation am Multifermenter geht erneut ein Optimierungsversuch mittels SDR voraus. In diesem Vorversuch werden, in Anlehnung an Beispiel 1, verschiedene NaHCO₃-Konzentrationen in Kombination mit den Medien Sod-ß / H und TES / K getestet. Bei dem Experiment wird zusätzlich zur Beurteilung mittels pO₂-Abfall eine identisch belegte Wellplatte mit pH-Sensoren durchgeführt. Die Ergebnisse dienen der Entscheidungsfindung für das aktuelle Beispiel, die aussichtsreichsten Kombinationen (TES / K + NaHCO₃ / D und Sod-ß / H + NaHCO₃ / E) werden in den Experimentplan aufgenommen. (Die Daten aus dem Vorversuch sind hier nicht gezeigt.)

Es wird in zwei Multifermenter (entspricht zwölf Einzelfermentern) mit unterschiedlichen Medienkombinationen fermentiert.

Die Durchführung und das Handling basiert auf den unter Materialien, Methoden und Arbeitschritte beschriebenen Punkten. Es folgt das Versuchssetup und der Experimentplan zur Übersicht in Tabelle 5.

| | | | | | |
|---|---|---|---|---|---|
| **Plattform:** | Bioreaktor/Multifermenter | **Zelle:** | CHO | **Laufzeit:** | 11 d (11 Tage) |
| **Medium:** | MEDIUM B w/o NaHCO₃ + Einwaage/Stocklösung Puffersubstanz(en) | | | | |
| **Feeding:** | Ab dx kontinuierlicher Feed | **T:** | 37,0°C | **Einsaatdichte:** | 1,7 |
| **CO₂-Profil:** | d0 bis d3: 8 % - d3 bis d6: 6 % - d6 bis d8: 2 % - d8 bis d11: 4 % | | | | |

Das obig gezeigte CO₂-Profil gilt für den geregelten Bioreaktor FS 33.1. Bei den restlichen Fermentern wurde nach der Einsaat (d0), für eine bestimmte Zeit bei einer bestimmten Stellgröße, CO_{2 (gas)} zugegeben um den Zellen ein Minimum an gelösten CO₂ anzubieten. An d1 wurden weitere pCO₂-Messungen durchgeführt, bei denen sich herausstellte, dass in den Kulturen im Schnitt ca. 2 bis 3 % CO₂ vorlagen. Im Kontext einer Zelldichtebestimmung wurde gemeinsam beschlossen, dass keine weiteren manuellen Eingriffe zur CO₂-Begasung mehr notwendig sind.

Das Regelverhalten wurde erneut beobachtet. Um später eine objektive Beurteilung durchführen zu können wurden dabei keine Einstellungen geändert, oder per Hand eingegriffen. Die Analyse des Regelverhaltens soll als Basis für die getrennte (zukünftig separater positiver und negativer Stellbereich) Regelereinstellung dienen.

**Tabelle 5 Experimentplan**

| **FS** | **Puffersubstanz (en) / Konzentration** | **pH-Agenzien** | **pCO₂-Management** |
|---|---|---|---|
| 32.1 | TES / K | CO₂/NaOH | Definierte Anfangsbegasung (d0) |
| 32.2 | TES / K + NaHCO₃ / D | CO₂/NaOH | Definierte Anfangsbegasung (d0) |
| 32.3 | TES / K + NaHCO₃ / D | CO₂/Na₂CO₃ | Definierte Anfangsbegasung (d0) |
| 32.4 | Sod-ß / H | CO₂/NaOH | Definierte Anfangsbegasung (d0) |
| 32.5 | Sod-ß / H + NaHCO₃ / E | CO₂/NaOH | Definierte Anfangsbegasung (d0) |
| 32.6 | TES / K | CO₂/NaOH | Keine Zugabe von CO₂ |
| 33.1 | TES / K | CO₂/NaOH | Automatische Regelung |
| 33.2 | Sod-ß / H + NaHCO₃ / E | CO₂/Na₂CO₃ | Definierte Anfangsbegasung (d0) |
| 33.3 | MEDIUM B (NaHCO₃ /F) (Vergl.-Kontrolle) | CO₂/NaOH | Definierte Anfangsbegasung (d0) |
| 33.4 | MEDIUM B (NaHCO₃ /F) (Kontrolle) | CO₂/Na₂CO₃ | Definierte Anfangsbegasung (d0) |
| 33.5 | MEDIUM B (NaHCO₃ /D) | CO₂/NaOH | Definierte Anfangsbegasung (d0) |
| 33.6 | MEDIUM B (NaHC0₃ /D) | CO₂/Na₂CO₃ | Definierte Anfangsbegasung (d0) |

Die Fermentationssysteme (FS) 32.1, 32.6 und 33.6 fielen über den Fermentationszeitraum hinweg leider auf Grund von Kontaminationen aus. Die daraus erhobenen Daten fließen nicht, oder nur beschränkt, in die Ergebnisbetrachtungen ein.

### Vergleich pCO₂ geregelt gegen pCO₂ ungeregelt

Da sowohl FS 32.1 als auch FS 32.6, welche wie der geregelte Bioreaktor FS 33.1 mit dem Zellkulturmedium TES / K betrieben werden, wegen Kontamination ausfiel kann der Unterschied nur abschätzend durch Vergleich mit FS 32.2 und FS 32.3 (beide TES / K + NaHCO₃ / D) verglichen werden.

Abbildung 4A und B zur normalen Lebendzellzahlkonzentration (A) bzw. Titerkonzentration (B) zeigt, dass die zellspezifische Produktbildungsrate des geregelten Fermentationslaufes (Kurve mit Dreiecksymbol) höher ist als diejenige des vergleichbaren ungeregelten Fermentationslaufes (Kurve mit Raute).

Weiterhin zeigen die Versuche aus Abbildung 4, dass die pH-Regelung mit NaOH tendenziell eine bessere Produktbildungsrate aufweist als diejenige mit Na₂CO₃.

Bei einer weiteren Optimierung des pCO₂ Profils wird die spezifische Produktbildungsrate weiter erhöht.

Die Lebendzellzahldichte des pCO₂-geregelten Laufs (FS 33.1) weicht, wie in Abbildung 4B ersichtlich um maximal 20 Einheiten voneinander ab. Ab d10 streuen auch die beiden Vergleichsläufe gering, sonst wird ist eine gute Übereinstimmung erzielt. Das niedrigere Niveau von FS 33.1 kann aus dem höheren pCO₂-Profil resultieren und ist nicht negativ zu interpretieren. Die Vitalität (nicht gezeigt) ist bei allen Läufen auf gleichem Niveau.

Die Laugenverbräuche (nicht gezeigt) sind bei allen Läufen gering. Bei FS 32.2 (höchste Lebendzellzahlkonzentration) muss gegen Ende der Fermentation (d9-d11) erwartungsgemäß mehr Lauge zugegeben werden. Trotzdem weißt dieser Lauf am Fermentationsende mit 393 mosmol/kg die geringste Osmolalität auf (nicht gezeigt.)

In Abbildung 4A ist ersichtlich, dass FS 33.1 trotz geringerer Zellzahl als FS 32.3 eine höhere Titerkonzentration aufweißt. Die Konzentration liegt im oberen Drittel der Streuung zwischen den beiden identischen Fermentationsläufen FS 32.2 und FS 32.3. Das heißt, dass der Fermentationslauf mit der pCO₂-Regelung eine höhere spezifische Produktbildungsrate besitzt.

### Vergleich Puffersysteme

Im Anschluss erfolgt der Vergleich der unterschiedlichen Puffersysteme gegeneinander.

Abbildung 5A zeigt, dass sich das pCO₂ Profil des geregelten Fermentationslaufes (FS33.1) deutlich von den Vergleichsfermentationen (FS32.2 und FS32.3) abhebt. FS32.2 und FS32.3 werden zum Vergleich herangezogen obwohl sie einen geringen Anteil von NaHC0₃ aufweisen, da aufgrund einer Kontamination ein identischer Vergleichspartner entfallen ist. Das durch die Regelung erzielte pCO₂ Profil hebt sich im ersten Drittel der Fermentation deutlich von den Vergleichsläufen ab und folgt der Sollvorgabe (siehe Abbildung 5B, d1-d3 pCO₂ = 8%, d3-d6 pCO₂ = 6%, d6-d8 pCO₂ = 2% und von d8-d11 pCO₂ = 4%) weitestgehend. Beim Erzwingen eines niedriger als normalen pCO2 Wertes (Tag 6-8, d6-d8) wird Stickstoff eingegast um CO₂ auszutreiben. Es ist festzustellen, dass der niedrige Sollwert nie erreicht wird und ein pCO₂ Wert von 3.2% eine kritische Untergrenze darstellt für die Regelung. Bei der dabei erhöhten Anforderung von Stickstoff wird im Zusammenhang auch Sauerstoff ausgetrieben, welcher durch einen höheren Sauerstoffvolumenstrom ausgeglichen wird. Es kann einer kritischen Gesamtgasmenge erreicht werden, bei welcher eine unverhältnismäßig große Schaumbildung festzustellen ist.

Aus Abbildung 5B ist ersichtlich, dass sich der geregelte Fermentationslauf auch von allen anderen Vergleichsläufen überraschend deutlich abhebt. Das heißt, dass das durch die Regelung eingestellte pCO₂ Profil tatsächlich auf der Sollvorgabe (d1-d3 pCO₂ = 8%, d3-d6 pCO₂ = 6%, d6-d8 pCO₂ = 2% und von d8-d11 pCO₂ = 4%) beruht und nicht zufällig zustande kommt.

Abbildung 5C zeigt, dass die mit TES ohne NaHCO₃ bzw. TES mit 8mmol/L NaHCO₃ erreichten Titerkonzentrationen vergleichbar sind mit der Kontrolle (NaHCO₃ basierter Puffer mit einer Konzentration von 36mmol/L). Der Lauf mit TES ohne NaHCO₃ (geregelt) erreicht trotz zufällig gewählten und nicht optimierten pCO2 Profil eine Endkonzentration (Titer), welche sich um lediglich 6.10% (gemessen an der Kontrolle) von der Kontrolle unterscheidet. Der Lauf mit TES plus 8mmol/L NaHCO₃ (ungeregelt) erreicht eine Endkonzentration (Titer), welche sich um lediglich 0.01%

(gemessen an der Kontrolle) von der Kontrolle unterscheidet. Dies zeigt vollkommen überraschend, dass NaHCO₃ vollständig durch TES ersetzt werden kann. Weiterhin zeigt dies, dass das Ergebnis des Laufs mit TES plus 8mmol/L NaHCO₃ (ungeregelt) die gleiche Endtiterkonzentration erreicht wie die Kontrolle.

Optimalerweise läuft ein Fermentationslauf unter folgenden Bedingungen ab: TES-Puffer komplett ohne NaHCO₃ und mit pCO₂ Regelung. Hiermit werden die besten Endtiter erreicht.

### BEISPIEL 4 FERMENTATION VERIFIZIERUNG PUFFERSYSTEM

In sechs CO₂-geregelten Bioreaktoren werden die Puffersysteme TES und Sod-ß mit unterschiedlich hohen Konzentrationen an NaHCO₃ eingesetzt, um die optimale Konzentration an NaHCO₃ im Medium zu bestimmen. Im Folgenden wird das Versuchssetup dargestellt:

| | |
|---|---|
| Plattform: | Bioreaktor / Multifermenter |
| Verfahren: | Fed Batch |
| Zelle: | CHO |
| Einsaatdichte: | 3x10⁵ vc/mL |
| Startvolumen: | 1,8 L |
| Laufzeit: | 11 Tage |
| Medium: Medium | B, NaHCO₃-frei, MOPS-frei + Einwaage/Stocklösung Puffersubstanz(en) |
| Feeding: | Feed I kontinuierlich ab Tag 2 |
| Temperatur: | 37°C |
| pO₂: | 60 % |
| Drehzahl: | 160 Upm |
| pH: | Tag 0-36,95 ± 0,15, Tag 3-116,80 ± 0,15 |
| pH-Regelung: | NaOH (1,2M) (kein CO₂) |
| pCO₂: | Tag 0-3 5%, Tag 3-11 3% |

Die eingesetzten Puffersysteme mit unterschiedlich hohen Konzentrationen an NaHCO₃ sind in Tabelle 6 dargestellt.

**Tabelle 6 Experimentplan mit eingesetzten Puffersystemen und NaHCO₃-Konzentrationen.**

| **Lauf** | **Puffersubstanz (Konzentration)** | **Konzentration an NaHCO₃ [M]** |
|---|---|---|
| 1.1 | TES (40mM) | - |
| 1.2 | TES (40mM) | 8 |
| 1.3 | Sod-ß (25mM) | - |
| 1.4 | Sod-ß (25mM) | 8 |
| 1.5 | Sod-ß (25mM) | 12 |
| 1.6 | Sod-ß (25mM) | 16 |

Die in Abbildung 6 dargestellten normierten Produktkonzentrationen weisen bei Einsatz der gleichen Puffersubstanz keine Unterschiede im Hinblick auf die eingesetzte NaHCO₃-Konzentration auf. Mit Einsatz von TES (1.1, 1.2) ergeben sich durch unterschiedlich hohe NaHCO₃-Konzentrationen gleiche Produktkonzentrationen. Auch in den restlichen Fermentationen mit Einsatz von Sod-ß (1.3, 1.4, 1.5, 1.6) und verschiedenen Konzentrationen an NaHCO₃ bestätigt sich die Aussage, dass die Produktivität der Zellen unabhängig von der eingesetzten NaHCO₃-Konzentration im Medium ist. Aus diesem Grund ist es möglich Zellen ohne den Einsatz von NaHCO₃ zu kultivieren.

### BEISPIEL 5 FERMENTATION VERGLEICH CO₂-PROFILE

In fünf CO₂-geregelten Bioreaktoren werden über die Kultivierungsdauer hinweg verschiedene CO₂-Sollwerte vorgegeben, um das optimale CO₂-Profil für einen Fermentationsprozess zu finden.

Im Folgenden wird das Versuchssetup dargestellt:

| | |
|---|---|
| Plattform: | Bioreaktor / Multifermenter |
| Verfahren: | Fed Batch |
| Zelle: | CHO |
| Einsaatdichte: | 3x10⁵ vc/mL |
| Startvolumen: | 1,8 L |
| Laufzeit: | 11 Tage |
| Medium: | Medium B, NaHCO₃-frei, MOPS-frei + Sod-ß (25mM) |
| Feeding: | Feed I kontinuierlich ab Tag 2 |
| Temperatur: | 37°C |
| pO₂: | 60 % |
| Drehzahl: | 160 Upm |
| pH: | Tag 0-36,95 ± 0,15, Tag 3-116,80 ±0,15 |
| pH-Regelung: | NaOH (1,2M) (kein CO₂) |

Die unterschiedlichen CO₂-Profile der sechs Fermentationsprozesse sind in Tabelle 7 dargestellt.

**Tabelle 7 Experimentplan mit verschiedenen CO₂-Profilen.**

| **Lauf** | **CO₂-Profil** |
|---|---|
| 3.1 | Tag 0-3 5% CO₂, Tag 3-11 3% CO₂ |
| 3.2 | Tag 0-2 8% CO₂, Tag 2-11 6% CO₂ |
| 3.3 | Tag 0-4 8% CO₂, Tag 4-8 10% CO₂, Tag 8-11 8% CO₂ |
| 3.4 | Tag 0-1 8% CO₂, Tag 1-2 6% CO₂, Tag 2-7 8% CO₂, Tag 7-11 5% CO₂ |
| 3.5 | Tag 0-11 3% CO₂ |

Da an Tag 0 versäumt wurde am Nachmittag erneut eine Probe für eine CO₂-Korrektur zu entnehmen, stimmen die vorgegebenen CO₂-Werte (siehe Tabelle 6) von Tag 0 bis 1 nicht mit den gemessen Werten überein wie in Abbildung 7C ersichtlich ist.

Sowohl die normierten Lebendzellkonzentrationen als auch Produktkonzentrationen der einzelnen Prozesse mit unterschiedlichen CO₂-Profilen ähneln sich weitgehend bis auf den Lauf 3.2. In dem Prozess 3.2 ist das Wachstum der Zellen schlechter als in allen anderen Prozessen (siehe Abbildung 7A). Durch das schlechte Wachstum der Zellen und die niedrige Zelldichte bleibt auch die Produktkonzentration in diesem Lauf niedriger als bei den anderen (siehe Abbildung 7B). Im Bezug auf das CO₂-Profil des Laufes 3.2 ist in Abbildung 7C ersichtlich, dass dies der einzige Lauf ist, der an Tag 1 einen hohen pCO₂ (> 8%) aufweist.

Somit wird das Wachstum der Zellen durch einen anfänglich hohen pCO₂ negativ beeinflusst.

### BEISPIEL 6 SCHÜTTELKOLBEN SÄUREVERTRÄGLICHKEIT MIT EINSATZ DER NA-SALZE

In 10 250-mL-Schüttelkolben werden Zellen auf ihre Säureverträglichkeit mit Zugabe von Natriumsalzen getestet. Dabei werden die Salzlösungen so gewählt, dass sich die in einer vorhergehenden Titration ermittelten Stoffmengen der Säuren für einen pH-Shift von 7,1 nach 6,7 in 1,5 mL Lösung befinden. An Tag 3 werden den Schüttelkolben diese 1,5 mL der verschiedenen Salzlösungen zugegeben, um einen pH-Shift zu simulieren.

Im Folgenden wird das Versuchssetup dargestellt:

| | |
|---|---|
| Kulturgefäß/Füllmeng: | 250mL/50mL |
| Zelle: | CHO |
| Medium: | Medium B, NaHCO₃-frei, MOPS-frei + Sod-ß (25mM) |
| Einsaatdichte: | 2x10⁵ vc/mL |
| Kultivierungsdauer: | 11 Tage |
| Kultivierungsbedingungen: | Temperatur: 37°C |
| | Relative Feuchte: 70% |
| | Drehzahl: Tag 0-3 120 rpm, Tag 3-7 140 rpm |
| | CO₂: Tag 0-3 5%, Tag 3-11 3% |
| Feeding: | Feed I täglich 1,5 mL |

Die unterschiedlichen Zugaben in den 10 Schüttelkolben sind in Tabelle 8 dargestellt.

**Tabelle 8**

| **Schüttelkolben** | **Zugabe** |
|---|---|
| SF1 | Wasser (für Kontrolle) |
| SF2 | |
| SF3 | Natriumchlorid 23,4 g/L (für Salzsäure) |
| SF4 | |
| SF5 | Natriumacetat 39,4 g/L (für Essigsäure) |
| SF6 | |
| SF7 | Natriumsulfat 34,1 g/L (für Schwefelsäure) |
| SF8 | |
| SF9 | Di-Natriumhydrogenphosphat 61,7 g/L (für Phosphorsäure) |
| SF10 | |

Die in Abbildung 8 dargestellten normierten Lebendzellkonzentrationen zeigen, dass die Zellen in allen Schüttelkolben außer den Schüttelkolben SF 5 und 6 mit Zugabe von Natriumacetat ähnlich wachsen. In diesen Schüttelkolben werden niedrigere Zellkonzentrationen erreicht und die Zellen sterben bereits ab Tag 7, sodass die Zugabe von Natriumacetat ein wesentlich schlechteres Wachstum zur Folge hat.

Somit kann der Einsatz von Essigsäure zur pH-Regelung im Bioreaktor ausgeschlossen werden.

### BEISPIEL 7 FERMENTATION VERGLEICH SÄUREN

In drei CO₂-geregelten Bioreaktoren werden für einen pH-Shift von 6,95 nach 6,8 an Tag 3 verschiedene Säuren zugegeben. Dafür werden die Konzentrationen der Säurelösungen so gewählt, dass sich die notwendigen Stoffmengen, die zuvor in einer Titration ermittelt wurden, in 50 mL Lösung befinden.

Im Folgenden wird das Versuchssetup dargestellt:

| | |
|---|---|
| Plattform: | Bioreaktor / Multifermenter |
| Verfahren: | Fed Batch |
| Zelle: | CHO |
| Einsaatdichte: | 3x10⁵ vc/mL |
| Startvolumen: | 1,8 L |
| Laufzeit: | 11 Tage |
| Medium: | Medium B, NaHCO₃-frei, MOPS-frei + Sod-ß (25mM) |
| Feeding: | Feed I kontinuierlich ab Tag 2 |
| Temperatur: | 37°C |
| pO₂: | 60 % |
| Drehzahl: | 160 Upm |
| pH: | Tag 0-3 7,00 ± 0,05, Tag 3-11 6,80 ± 0,05 |
| pH-Regelung: | NaOH (1,2M) (kein CO₂) |

Die unterschiedlichen Zugaben der drei Fermentationsprozesse sind in Tabelle 9 dargestellt.

**Tabelle 9 Experimentplan mit verschiedenen Zugaben.**

| **Lauf** | **Zugabe** |
|---|---|
| 4.3 | Salzsäure 99mM |
| 4.4 | Schwefelsäure 54mM |
| 4.5 | Phosphorsäure 818mM |

Die normierten Zellkonzentrationen in Abbildung 9A verlaufen weitgehend ähnlich, sodass an Tag 10 die Maxima der einzelnen Läufe erreicht werden. Dabei bewegen sich die Zellkonzentrationen des Laufes 4.3 mit Zugabe von Salzsäure ständig unterhalb der anderen.

Im Hinblick auf die normierten Produktkonzentrationen (siehe Abbildung 9B) fällt jedoch auf, dass diese alle ähnlich verlaufen und somit im Lauf 4.3 mit Zugabe von Salzsäure gleiche Produktkonzentrationen erhalten werden wie in den anderen Prozessen.

Im Bezug auf die in Abbildung 9C dargestellten Laktatkonzentrationen wird deutlich, dass durch die Zugabe von Schwefelsäure (4.4) im Fermentationsprozess weniger Laktat produziert wird.

Damit ergibt sich durch die Zugabe der Salzsäure eine höhere Produktivität der Zellen und durch die Zugabe der Schwefelsäure ergeben sich niedrigere Laktatkonzentrationen im Fermentationsprozess.

### BEISPIEL 8 VERGLEICH STANDARDOPROZESS MIT CO₂-GEREGELTEM PROZESS

In vier Bioreaktoren wird der Standardprozess mit dem herkömmlichen Medium B ohne CO₂-Regelung und mit Einsatz von Na₂CO₃ zur pH-Regelung mit dem CO₂-geregelten Prozess mit dem Medium B, NaHCO₃-frei, MOPS-frei + Sod-ß (25mM) und Einsatz von NaOH und verschiedener Säuren für die pH-Regelung verglichen.

Im Folgenden wird das Versuchssetup dargestellt:

| | |
|---|---|
| Plattform: | Bioreaktor / Multifermenter |
| Verfahren: | Fed Batch |
| Zelle: | CHO |
| Einsaatdichte: | 3x10⁵ vc/mL |
| Startvolumen: | 1,8 L |
| Laufzeit: | 11 Tage |
| Feeding: | Feed I kontinuierlich ab Tag 2 |
| Temperatur: | 37°C |
| pO₂: | 60 % |
| Drehzahl: | 160 Upm |
| pH: | Tag 0-37,00 ± 0,05, Tag 3-11 6,80 ± 0,05 |

Die unterschiedlichen Zugaben der drei Fermentationsprozesse sind in Tabelle 10 dargestellt.

**Tabelle 10 Experimentplan mit verschiedenen Zugaben.**

| **Lauf** | **Medium** | **pH-Regelung** | **Säurezugabe** |
|---|---|---|---|
| 4.1 | Medium B | Na₂CO₃ | - |
| | | CO₂ | |
| 4.3 | Medium B, NaHCO₃-frei, MOPS-frei + Sod-ß (25mM) | NaOH | Salzsäure 99mM |
| | | Säurezugabe | |
| 4.4 | Medium B, NaHCO₃-frei, MOPS-frei + Sod-ß (25mM) | NaOH | Schwefelsäure 54mM |
| | | Säurezugabe | |
| 4.5 | Medium B, NaHCO₃-frei, MOPS-frei + Sod-ß (25mM) | NaOH | Phosphorsäure 81mM |
| | | Säurezugabe | |

Die in Abbildung 10 dargestellten normierten Produktkonzentrationen der Läufe 4.1 und 4.3 - 4.5 verlaufen sehr ähnlich. Dabei werden im Standardprozess (4.1) und im Lauf 4.5 mit Zugabe von Phosphorsäure annähernd gleiche Produktkonzentrationen erreicht, sodass der CO₂-geregelte Prozess keine Nachteile in Hinsicht auf die Produktivität oder die zu erreichende Produktkonzentration mit sich bringt.

### BEISPIEL 9 VERGLEICH CO₂ PROFILE MIT MODELLZELLE 2

In fünf Bioreaktoren wird der CO₂-geregelte Prozess mit dem Medium B, NaHCO₃-frei, MOPS-frei + Sod-ß (25mM) und Einsatz von NaOH für verschiedene pCO₂-Profile verglichen. Die Regelung des pCO₂ geschieht aktiv durch Begasung mit N₂ und CO₂, was durch die Entkoppelung der pH-Regelung möglich ist.

Im Folgenden wird das Versuchssetup dargestellt:

| | |
|---|---|
| Plattform: | Bioreaktor / Multifermenter |
| Verfahren: | Fed Batch |
| Zelle: | CHO |
| Einsaatdichte: | 3x10⁵ vc/mL |
| Startvolumen: | 1,8 L |
| Laufzeit: | 11 Tage |
| Feeding: | Feed I kontinuierlich ab Tag 2 |
| Temperatur: | 37°C |
| pO₂: | 60 % |
| Drehzahl: | 160 Upm |
| pH: | Tag 0-3 7,00 ± 0,05, Tag 3-11 6,80 ± 0,05 |

Die unterschiedlichen pCO₂-Profile der fünf Fermentationsprozesse sind in Tabelle 11 dargestellt.

**Tabelle 11 Experimentplan mit unterschiedlichen pCO₂-Profilen.**

| **Lauf** | **Tag 0 bis Tag3** | **Tag3 bis Tag7** | **Tag7 bis Tag11** |
|---|---|---|---|
| KF1 | 3% | 3% | 3% |
| KF2 | 7% | 3% | 3% |
| KF3 | 11% | 3% | 3% |
| KF4 | 7% | 11% | 7% |
| KF5 | 7% | 11% | 3% |

Die in Abbildung 11A dargestellten normierten Lebendzellkonzentrationen zeigen ein signifikant besseres Wachstumsverhalten für den Fermentationslauf KF1. Die Abbildung 11B für die normierte Produktkonzentration weist ebenfalls ein besseres Resultat für KF1 aus. Die Fermentationslaufzeit wurde für diesen Versuch in 3 Abschnitte unterteilt, für welche die Sollwerte des pCO₂ variiert wurden. Das besste Ergebnis wurde mit einer durchgängig niedrigen Vorgabe für den pCO₂ von 3% erzielt.

### ABKÜRZUNGEN

Im Folgenden werden in dieser Arbeit verwendete Abkürzungen und Symbole kurz erläutert.
- BI: Firma Boehringer Ingelheim GmbH & Co. KG
- CHO-Zelle: Chinese Hamster Ovarie - Zelle (Engl. "Zelle aus dem Eierstock des chinesischen Hamsters"). Eine seit 1960 etablierte und seither gut definierte und robuste Zelllinie die inzwischen auch in Suspension kultiviert werden kann und oft in der Pharmaproduktion Einsatz findet.
- DoE: Design of Experiments. Versuchsplanung zur Optimierung auf mathematisch-statistischer Grundlage. Software: Modde
- Downstream: Bereich der Produktisolierung und Anreicherung ab dem Zeitpunkt der Zellernte. (Volumenabnahme/Aufkonzentration).
- Feeding: (Engl. "Fütterung") Zugabe einer Lösung in die Fermentationskultur die von den Zellen zum Wachstum oder zur Proliferation benötigt wird.
- FS: Fermenter-Steuerplatz oder Fermentationssystem
- Gl.: Gleichung
- GZZ: Gesamtzellzahl. Konzentrationsangabe. Anzahl an Zellen pro Volumen die analysiert worden ist; beinhaltet sowohl tote als auch lebende Zellen. Meist Zelldichte in [Y Zellen x10⁵ / ml].
- IPC: In-Prozess-Kontrolle. Analytik von Parametern während ein Prozess abläuft. Kann Grundlage für Eingriffe in den laufenden Prozess sein wie z.B. Boluszugaben.
- LAF-Box: Laminar-Air-Flow-Box (Engl. "Mit laminarem Luftstrom durchströmte Einrichtung). Steriler Arbeitsbereich.
- LZZ: Lebendzellzahl. Konzentrationsangabe. Anzahl an Zellen pro Volumen die als lebend analysiert ist. Meist Zelldichte in [Y Zellen x10⁵ / ml] Entspricht GZZ minus Anzahl toter Zellen.
- MFC: Massendurchflussregler (Engl. Mass Flow Controller)
- MSR: Messen-Steuern-Regeln; Mess- und Regeltechnik
- Multifermenter: Ein sog. Multifermenter ist eine Zusammenfassung aus sechs baugleichen Einzelfermentern, weiteres siehe Materialien.
- NaHCO₃: Natriumhydrogencarbonat, auch (Natrium-) bicarbonat od. Natron
- PBS: (Engl. "Phosphate buffered saline"). Phosphat-gepufferte Salzlösung die isotonische Eigenschaften hat.
- Prozessformat: Unter einem Prozessformat versteht man die Festlegung der Prozessparameter. Es enthält ebenso zeitliche Angaben als auch Volumenströme von Feedings, Rührerdrehzahlen etc.
- Px: P steht für eine Puffersubstanz. PA steht z.B. für Puffersubstanz A. Bei Wiederholungen des Puffers PA1, PA2, PA3 usw.
- Scale-up: (Engl. "Maßstäbliche Vergrößerung") = Skalierung bzw. Skalierbarkeit Maßstabsvergrößerung einer Produktionsanlage; meist mit Hilfe von dimensionslosen Kennzahlen durchgeführt.
- Shakeflask (SF): (Engl. Schüttelkolben). Einweg-Kolben aus Kunststoff. Der Deckel ist mit einem Gasdurchlässigen Sterilfilter versehen und wird bei Probenzug und Feeding abgeschraubt.
- SDR: SensorDish Reader® Online-Datenerfassung aus spezieller Wellplatte mit Messdots. Nicht invasives Tool der Firma Presens, Regensburg.
- Titer: Gibt die Konzentration eines Produktes, z.B. eines Proteins wie z.B. eines Antikörpers in [mg/L] an. Nicht zu verwechseln mit dem medizinischen Titer im eigentlichen Sinn.
- Upstream: Bereich der Zellkulturtechnik beginnend mit der Inokulation des Fermenters und Fermentation bis hin zur Zell-Ernte. (Volumenzunahme).
- WFI: Water for injection (Engl. "Wasser für Injektionszwecke") Aufgereinigtes, filtriertes, entsalztes Wasser dessen Qualität ständig überwacht wird.
- with: Ein Medium X with NaCl ist z.B. ein Medium bestehend aus allen Komponenten für Medium X -mit zusätzlicher definierter Menge NaCl.
- w/o: Im Bereich der Medienherstellung gängige Abkürzung für engl. "without". Ein Medium X w/o NaCl ist z.B. ein Medium bestehend aus allen Komponenten für Medium X -aber ohne NaCl.
- (aq): Index In wässriger Lösung
- d: Tag Fermentationstag. d0 = Tag der Einsaat.

## Patentansprüche

1. Eine Methode zur Optimierung eines biopharmazeutischen Produktionsprozesses umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponente Sodium-β-glycerophosphat-pentahydrat (C₃H₇Na₂O₆P x 5 H₂O, Sod-ß) enthält,
c) Regulierung des pH-Wertes über eine nicht CO₂-bildende Säure und/oder Lauge,
wobei während des Bioprozesses eine Regulierung des pCO₂ mit CO₂, O₂, N₂ und/oder Luftzufuhr oder mittels der Drehzahl des Rührers durchgeführt wird.

2. Eine Methode zur Regulierung des pCO₂ umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponente Sodium-β-glycerophosphat-pentahydrat (C₃H₇Na₂O₆P x 5 H₂O, Sod-ß) enthält,
c) Regulierung des pH-Wertes über eine nicht CO₂-bildende Säure und/oder Lauge,
wobei während des Bioprozesses eine Regulierung des pCO₂ mit CO₂, O₂, N₂ und/oder Luftzufuhr oder mittels der Drehzahl des Rührers durchgeführt wird.

3. Eine Methode zur Verbesserung der Reproduzierbarkeit von Bioprozessen mit eukaryotischen Zellen dadurch charakterisiert, dass mindestens folgende Parameter kontrolliert werden: pCO2 Profil, O₂ Profil, pH-Profil, Temperaturprofil, Drehzahl und
a) wobei die Kultivierung der Zelle im Fed-Batch Fermentationsverfahren in einem Zellkulturmedium erfolgt, welches keine HCO₃⁻ bzw. CO₃²⁻ Ionen enthält, und
b) wobei besagtes Zellkulturmedium die Pufferkomponente Sodium-β-glycerophosphatpentahydrat (C₃H₇Na₂O₆P x 5 H₂O, Sod-ß) enthält,
c) wobei die Regulierung des pH-Wertes über eine nicht CO₂-bildende Säure und/oder Lauge erfolgt und
d) wobei während des Bioprozesses eine Regulierung des pCO₂ mit CO₂, O₂, N₂ und/oder Luftzufuhr oder mittels der Drehzahl des Rührers durchgeführt wird.

4. Eine Methode zur Herstellung eines rekombinanten Produkts von Interesse umfassend folgende Schritte:
a) Bereitstellung einer eukaryotischen Wirtszelle, welche ein rekombinantes Gen von Interesse enthält und ein korrespondierendes Produkt von Interesse produziert,
b) Kultivierung der Zelle aus Schritt a) im Fed-Batch Fermentationsverfahren in einem Zellkulturmedium, welches keine HCO₃⁻ bzw. CO₃²⁻ Ionen enthält, wobei besagtes Zellkulturmedium die Pufferkomponente Sodium-β-glycerophosphat-pentahydrat (C₃H₇Na₂O₆P x 5 H₂O, Sod-ß) enthält,
c) Regulierung des pH-Wertes über eine nicht CO2-bildende Säure und/oder Lauge,
wobei während des Bioprozesses eine Regulierung des pCO₂ mit CO₂, O₂, N₂ und/oder Luftzufuhr oder mittels der Drehzahl des Rührers durchgeführt wird.

5. Die Methode gemäß Anspruch 4 **dadurch gekennzeichnet, dass** das Produkt von Interesse ein Protein ist, bevorzugt ein Antikörper oder Antikörperfragment oder Fc-Fusionsprotein.

6. Die Methode nach einem der Ansprüche 1-5 dadurch charakterisiert, dass eine Regulierung des pCO₂ ausschließlich durch Begasung mit CO₂ und/ oder N₂ erfolgt.

7. Die Methode nach einem der Ansprüche 1-6 dadurch charakterisiert, dass die Sod-ß Konzentration zwischen 1mmol/L bis 100mmol/L, 5-50, 15-30 bzw. kleiner gleich 100mmol/L bzw. 25mmol/L beträgt.

8. Die Methode nach einem der Ansprüche 1-7 dadurch charakterisiert, dass die Säure und/ oder Lauge gemäß Schritt c) NaOH, HCl, H₃PO₄ oder H₂SO₄ ist, bevorzugt NaOH oder HCl.

9. Die Methode nach einem der Ansprüche 1-8 dadurch charakterisiert, dass die eukaryotische Zelle eine Säugerzelle, bevorzugt eine Nagerzelle, bevorzugt eine CHO, PER.C6 oder NSO Zelle ist.

10. Die Methode nach einer der Ansprüche 1 bis 9 dadurch charakterisiert, dass der pCO₂ in der Anwachsphase (Tag 0 bis Tag 3 bzw. Tag 1 bis 3, insbesondere an Tag 1 der Fermentation) auf einen Wert kleiner oder gleich 10%, bzw. kleiner oder gleich 8% reguliert wird, bevorzugt wird der pCO₂ eingestellt auf einen Wert zwischen 2% und 8% bzw. zwischen 3% und 8% , bzw. zwischen 5% und 8% , besonders bevorzugt ist ein pCO₂ Wert von 5% bzw. 3%.

11. Die Methode nach einer der Ansprüche 1 bis 10 dadurch charakterisiert, dass der pCO₂ von Tag 0 bis Tag 11 (bzw. bis Fermentationsende) auf 3% reguliert wird.

12. Die Methode nach Anspruch 10 oder 11 dadurch charakterisiert, dass der pCO₂ Wert zusätzlich wie folgt reguliert wird:
i) ein mittlerer pCO₂ (kleiner oder gleich 12%) in der Wachstumsphase (Tag 3-7 bzw. Tag 4-8), bevorzugt 5-12% bzw. 8-12%, besonders bevorzugt 8-10%, bzw. 5-11%, bzw. 3-11%,
ii) ein leicht erhöhter bzw. hoher pCO₂ (größer oder gleich 5%, 8% bzw. 15%) in der Absterbephase (Tag 7-11 bzw. Tag 9-11 bzw. bis zum Ende der Fermentation), bevorzugt 15-18% bzw. 5-10% bzw. 15%.

13. Ein Zellkulturmedium, welches keine HCO₃⁻ bzw. CO₃²⁻ Ionen enthält, wobei besagtes Zellkulturmedium die Pufferkomponente Sodium-β-glycerophosphat-pentahydrat (C₃H₇Na₂O₆P x 5 H₂O, Sod-ß) enthält.

14. Das Zellkulturmedium nach Anspruch 13, dadurch charakterisiert, dass die Sod-ß Konzentration zwischen 1mmol/L bis 100mmol/L, 5-50, 15-30 bzw. kleiner gleich 100mmol/L bzw. 25mmol/L beträgt.

15. Ein Satz an Fed-Batch Fermentations Ausrüstungsteilen, auf englisch "kit", umfassend folgende Komponenten:
(a) Zellkulturmedium, welches komplett HCO₃⁻ bzw. CO₃²⁻ Ionen frei ist, wobei besagtes Zellkulturmedium die folgende Pufferkomponente enthält: Sodium-β-glycerophosphatpentahydrat (C₃H₇Na₂O₆P x 5 H₂O, Sod-ß),
(b) Fed-Batch Fermenter,
(c) eukaryontische Zelle, bevorzugt eine Säugerzelle, Nagerzelle, Hamsterzelle, insbesondere bevozugt eine CHO Zelle.

## Claims

1. A method for optimising a biopharmaceutical production process comprising the following steps:
a) preparing a eukaryotic host cell which contains a recombinant gene of interest and produces a corresponding product of interest,
b) cultivating the cell from step a) by the fed-batch fermentation process in a cell culture medium which contains no HCO₃⁻ or CO₃²⁻ ions, said cell culture medium containing the buffer component sodium-β-glycerophosphate-pentahydrate (C₃H₇Na₂O₆P x 5 H₂O, Sod-ß),
c) regulating the pH by means of a non-CO₂-forming acid and/or alkaline solution,
while during the bioprocess the pCO₂ is regulated with CO₂, O₂, N₂ and/or a supply of air or by means of the speed of the stirrer.

2. A method for regulating pCO₂, comprising the following steps:
a) preparing a eukaryotic host cell which contains a recombinant gene of interest and produces a corresponding product of interest,
b) cultivating the cell from step a) by the fed-batch fermentation process in a cell culture medium which contains no HCO₃⁻ or CO₃²⁻ ions, said cell culture medium containing the buffer component sodium-β-glycerophosphate-pentahydrate (C₃H₇Na₂O₆P x 5 H₂O, Sod-ß),
c) regulating the pH by means of a non-CO₂-forming acid and/or alkaline solution,
while during the bioprocess the pCO₂ is regulated with CO₂, O₂, N₂ and/or a supply of air or by means of the speed of the stirrer.

3. A method for improving the reproducibility of bioprocesses with eukaryotic cells, **characterised in that** at least the following parameters are monitored: pCO₂ profile, O₂ profile, pH-profile, temperature profile, speed and
a) wherein the cultivation of the cell by the fed-batch fermentation process takes place in a cell culture medium which contains no HCO₃⁻ or CO₃²⁻ ions, and
b) wherein said cell culture medium contains the buffer component sodium-β-glycerophosphate-pentahydrate (C₃H₇Na₂O₆P x 5 H₂O, Sod-ß),
c) wherein the pH is regulated by means of a non-CO₂-forming acid and/or alkaline solution, and
d) wherein during the bioprocess the pCO₂ is regulated with CO₂, O₂, N₂ and/or a supply of air or by means of the speed of the stirrer.

4. A method for preparing a recombinant product of interest comprising the following steps:
a) preparing a eukaryotic host cell which contains a recombinant gene of interest and produces a corresponding product of interest,
b) cultivating the cell from step a) by the fed-batch fermentation process in a cell culture medium which contains no HCO₃⁻ or CO₃²⁻ ions, said cell culture medium containing the buffer component sodium-β-glycerophosphate-pentahydrate (C₃H₇Na₂O₆P x 5 H₂O, Sod-ß),
c) regulating the pH by means of a non-CO₂-forming acid and/or alkaline solution,
wherein during the bioprocess the pCO₂ is regulated with CO₂, O₂, N₂ and/or a supply of air or by means of the speed of the stirrer.

5. The method according to claim 4, **characterised in that** the product of interest is a protein, preferably an antibody or antibody fragment or Fc-fusion protein.

6. The method according to one of claims 1-5, **characterised in that** the pCO₂ is regulated exclusively by the introduction of CO₂ and/ or N₂ gas.

7. The method according to one of claims 1-6, **characterised in that** the Sod-ß concentration is between 1 mmol/L and 100 mmol/L, 5-50, 15-30 or less than or equal to 100 mmol/L or 25 mmol/L.

8. The method according to one of claims 1-7, **characterised in that** the acid and/or alkaline solution according to step c) is NaOH, HCl, H₃PO₄ or H₂SO₄, preferably NaOH or HCl.

9. The method according to one of claims 1-8, **characterised in that** the eukaryotic cell is a mammalian cell, preferably a rodent cell, preferably a CHO, PER.C6 or NS0 cell.

10. The method according to one of claims 1 to 9, **characterised in that** the pCO₂ in the initial growth phase (day 0 to day 3 or day 1 to 3, particularly on day 1 of the fermentation) is regulated to a value of less than or equal to 10%, or less than or equal to 8%, preferably the pCO₂ is adjusted to a value between 2% and 8% or between 3% and 8%, or between 5% and 8%, a pCO₂ value of 5% or 3% being particularly preferred.

11. The method according to one of claims 1 to 10, **characterised in that** the pCO₂ from day 0 to day 11 (or to the end of the fermentation) is regulated to 3%.

12. The method according to claim 10 or 11, **characterised in that** the pCO₂ value is additionally regulated as follows:
i) an average pCO₂ (less than or equal to 12%) in the growth phase (day 3-7 or day 4-8), of preferably 5-12% or 8-12%, particularly preferably 8-10%, or 5-11%, or 3-11%,
ii) a slightly elevated or high pCO₂ (greater than or equal to 5%, 8% or 15%) in the dying-off phase (day 7-11 or day 9-11 or up to the end of the fermentation), of preferably 15-18% or 5-10% or 15%.

13. A cell culture medium which contains no HCO₃⁻ or CO₃²⁻ ions, said cell culture medium containing the buffer component sodium-β-glycerophosphate-pentahydrate (C₃H₇Na₂O₆P x 5 H₂O, Sod-ß).

14. The cell culture medium according to claim 13, **characterised in that** the Sod-ß concentration is between 1 mmol/L and 100 mmol/L, 5-50, 15-30 or less than or equal to 100 mmol/L or 25 mmol/L.

15. A set of items of fed-batch fermentation equipment, or kit, comprising the following components:
(a) cell culture medium which is completely free from HCO₃⁻ or CO₃²⁻ ions, said cell culture medium containing the following buffer component: sodium-β-glycerophosphate-pentahydrate (C₃H₇Na₂O₆P x 5 H₂O, Sod-ß),
(b) fed-batch fermenter,
(c) eukaryotic cell, preferably a mammalian cell, rodent cell, hamster cell, particularly preferably a CHO cell.

## Revendications

1. Méthode d'optimisation d'un procédé de production biopharmaceutique comprenant les étapes suivantes :
a) mise à disposition d'une cellule hôte eucaryote qui contient un gène recombinant d'intérêt et produit un produit d'intérêt correspondant,
b) culture de la cellule de l'étape a) dans un procédé de fermentation en lot dans un milieu de culture cellulaire qui ne contient pas d'ions HCO₃⁻ ou CO₃²⁻, ledit milieu de culture cellulaire contenant le composant tampon β-glycérophosphate-pentahydrate de sodium (C₃H₇Na₂O₆P x 5 H₂O, Sod-β),
c) régulation du pH par un acide et/ou une liqueur ne formant pas de CO₂,
dans laquelle pendant le bioprocessus, une régulation du pCO₂ est réalisée avec du CO₂, de l'O₂, du N₂ et/ou un apport d'air ou au moyen du nombre de tours de l'agitateur.

2. Méthode de régulation du pCO₂ comprenant les étapes suivantes :
a) mise à disposition d'une cellule hôte eucaryote qui contient un gène recombinant d'intérêt et produit un produit d'intérêt correspondant,
b) culture de la cellule de l'étape a) dans un procédé de fermentation en lot dans un milieu de culture cellulaire qui ne contient pas d'ions HCO₃⁻ ou CO₃²⁻, ledit milieu de culture cellulaire contenant le composant tampon β-glycérophosphate-pentahydrate de sodium (C₃H₇Na₂O₆P x 5 H₂O, Sod-β),
c) régulation du pH par un acide et/ou une liqueur ne formant pas de CO₂,
dans laquelle pendant le bioprocessus, une régulation du pCO₂ est réalisée avec du CO₂, de l'O₂, du N₂ et/ou un apport d'air ou au moyen du nombre de tours de l'agitateur.

3. Méthode d'amélioration de la reproductibilité de bioprocessus avec des cellules eucaryotes, **caractérisée en ce qu'**au moins des paramètres suivants sont contrôlés : le profil de pCO₂, le profil d'O₂, le profil du pH, le profil de température, le nombre de tours et
a) dans laquelle la culture de la cellule s'effectue dans un procédé de fermentation en lot dans un milieu de culture cellulaire qui ne contient pas d'ions HCO₃⁻ ou CO₃²⁻, et
b) dans laquelle ledit milieu de culture cellulaire contient le composant tampon β-glycérophosphatepentahydrate de sodium (C₃H₇Na₂O₆P x 5 H₂O, Sod-β),
c) dans laquelle la régulation du pH s'effectue par un acide et/ou une liqueur ne formant pas de CO₂, et
d) dans laquelle pendant le bioprocessus, une régulation du pCO₂ est réalisée avec du CO₂, de l'O₂, du N₂ et/ou un apport d'air ou au moyen du nombre de tours de l'agitateur.

4. Méthode de production d'un produit recombinant d'intérêt, comprenant les étapes suivantes :
a) mise à disposition d'une cellule hôte eucaryote qui contient un gène recombinant d'intérêt et produit un produit d'intérêt correspondant,
b) culture de la cellule de l'étape a) dans un procédé de fermentation en lot dans un milieu de culture cellulaire qui ne contient pas d'ions HCO₃⁻ ou CO₃²⁻, ledit milieu de culture cellulaire contenant le composant tampon β-glycérophosphate-pentahydrate de sodium (C₃H₇Na₂O₆P x 5 H₂O, Sod-β),
c) régulation du pH par un acide et/ou une liqueur ne formant pas de CO₂,
dans laquelle pendant le bioprocessus, une régulation du pCO₂ est réalisée avec du CO₂, de l'O₂, du N₂ et/ou un apport d'air ou au moyen du nombre de tours de l'agitateur.

5. Méthode selon la revendication 4, **caractérisée en ce que** le produit d'intérêt est une protéine, de préférence, un anticorps ou un fragment d'anticorps ou une protéine de fusion Fc.

6. Méthode selon l'une des revendications 1 à 5, **caractérisée en ce qu'**une régulation du pCO₂ s'effectue exclusivement par gazage avec du CO₂ et/ou du N₂.

7. Méthode selon l'une des revendications 1 à 6, **caractérisée en ce que** la concentration de Sod-β se situe entre 1 mmole/l à 100 mmoles/l, 5 à 50, 15 à 30 ou inférieure ou égale à 100 mmoles/l ou 25 mmoles/l.

8. Méthode selon l'une des revendications 1 à 7, **caractérisée en ce que** l'acide et/ou la liqueur selon l'étape c) est du NaOH, du HCl, du H₃PO₄ ou du H₂SO₄, de préférence, du NaOH ou du HCl.

9. Méthode selon l'une des revendications 1 à 8, **caractérisée en ce que** la cellule eucaryote est une cellule de mammifère, de préférence, une cellule de rongeur, de préférence une cellule CHO, PER.C6 ou NSO.

10. Méthode selon l'une des revendications 1 à 9, **caractérisée en ce que** le pCO₂ dans la phase de croissance (jour 0 à jour 3 ou jour 1 à jour 3, en particulier au jour 1 de la fermentation) est régulé à une valeur inférieure ou égale à 10 % ou inférieure ou égale à 8 %, de préférence, le pCO₂ est réglé à une valeur entre 2 % et 8 % ou entre 3 % et 8 % ou entre 5 % et 8 %, de manière particulièrement préférée, une valeur de pCO₂ est de 5 % ou 3 %.

11. Méthode selon l'une des revendications 1 à 10, **caractérisée en ce que** le pCO₂ du jour 0 au jour 11 (ou à la fin de la fermentation) est régulé à 3 %.

12. Méthode selon la revendication 10 ou 11, **caractérisée en ce que** la valeur de pCO₂ est en outre régulée comme suit :
i) un pCO₂ moyen (inférieur ou égal à 12 %) dans la phase de croissance (jour 3 à 7 ou jour 4 à 8), de préférence 5 à 12 %, de manière particulièrement préférée, 8 à 12 %, de manière particulièrement préférée, 8 à 10 % ou 5 à 11 % ou 3 à 11 %,
ii) un pCO₂ légèrement augmenté ou supérieur (supérieur ou égal à 5 %, 8 % ou 15 %) dans la phase d'extinction (jour 7 à 11 ou jour 9 à 11 ou jusqu'à la fin de la fermentation), de préférence 15 à 18 % ou 5 à 10 % ou 15 %)

13. Milieu de culture cellulaire qui ne contient pas d'ions HCO₃⁻ ou CO₃²⁻, ledit milieu de culture cellulaire contenant le composant tampon β-glycérophosphate-pentahydrate de sodium (C₃H₇Na₂O₆P x 5 H₂O, Sod-β).

14. Milieu de culture cellulaire selon la revendication 13, **caractérisé en ce que** la concentration de Sod-β se situe entre 1 mmole/l à 100 mmole/l, 5 à 50, 15 à 30 ou est inférieure ou égale à 100 mmoles/l ou 25 mmoles/l.

15. Ensemble d'éléments d'équipement de fermentation en lot, en anglais, « kit », comprenant les composants suivants :
(a) un milieu de culture cellulaire qui est complètement dépourvu d'ions HCO₃⁻ ou CO₃²⁻, ledit milieu de culture cellulaire contenant le composant tampon suivant : β-glycérophosphate-pentahydrate de sodium (C₃H₇Na₂O₆P x 5 H₂O, Sod-β),
(b) un fermentateur en lot,
(c) une cellule eucaryote, de préférence, une cellule de mammifère, une cellule de rongeur, une cellule de hamster, de manière particulièrement préférée, une cellule CHO.
